# EUROPEAN PATENT APPLICATION

(11) **EP 1 568 368 A1**
(43) Date of publication of application: **31.08.2005**
(21) Application number: 04090071.4
(22) Date of filing: 26.02.2004
(51) Int. Cl.: A61K 31/44, A61K 31/427, A61K 31/4439, A61K 31/517, A61P 35/00

(54) **Pharmaceutical combination comprising a CDK inhibitor and a VEGF receptor inhibitor**

(71) Applicant: Schering Aktiengesellschaft, 13342 Berlin (DE)
(72) Inventor: Siemeister, Gerhard, 13503 Berlin (DE)

(57) **Abstract**

Pharmaceutical combinations comprising at least one compound of general formula (I): 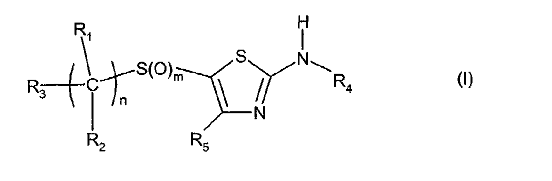 and at least one compound of general formula (II): 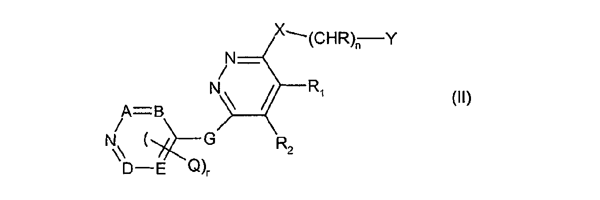 and/or of general formula (IIa): 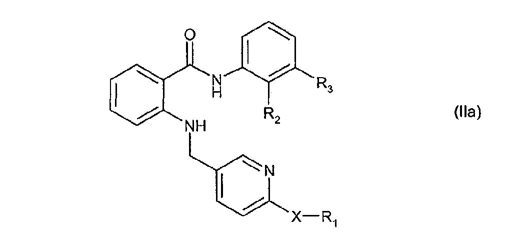 and/or of general formula (IIb): 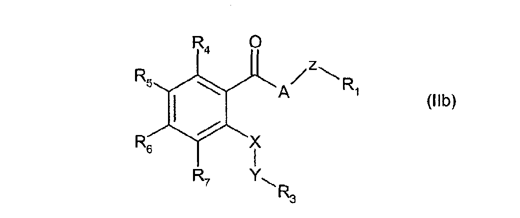 and their use for the treatment of different diseases.

## Description

The present invention concerns a pharmaceutical combination comprising a cyclin-dependent kinase (CDK) inhibitor and a vascular endothetial growth factor (VEGF) receptor inhibitor and the use of the combination for the treatment of different diseases.

The CDKs is an enzyme family of serine/threonine kinases that play an important role in the regulation of the cell cycle and is thus an especially advantageous target for the development of small inhibitory molecules. Selective inhibitors of the CDKs can be used for treating cancer or other diseases that disrupt cell proliferation.

The eukaryotic cell division ensures the duplication of the genome and its distribution to the daughter cells by passing through a coordinated and regulated sequence of events. The cell cycle is divided into four successive phases: the G1 phase represents the pre-DNA replication period in which the cell grows and is sensitive to external stimuli; the S phase the period during which the cell replicates its DNA; the G2 phase the period during which preparations are made for entry into mitosis; and finally, the Mitosis Phase (M phase) during which the replicated DNA separates and cell division is completed.

The CDKs require the binding of a cyclin (Cyc) as a regulatory subunit in order to activate and drive the cell through the cell cycle. Different CDK/Cyc pairs are active in the various phases of the cell cycle. CDK/Cyc pairs important for the basic function of the cell cycle are, for example, CDK4(6)/CycD, CDK2/CycE, CDK2/CycA, CDK1/CycA and CDK1/CycB. Some members of the CDK enzyme family have a regulatory function in that they influence the activity of the above-mentioned cell cycle CDKs without having a specific function which could be associated with other members of the CDK enzyme family. One of the latter, CDK5, is particular in that it has an atypical regulatory subunit (p35) that deviates from the cyclins, and in that its activity is highest in the brain.

The entry into the cell cycle and the passage through the "restriction point", which marks the independence of a cell from further growth signals for the completion of the cell division that has begun, are controlled by the activity of the CDK4(6)/CycD and CDK2/CycE complexes. The essential substrate of these CDK complexes is the retinoblastoma protein (Rb), a transcriptional co-repressor protein and the product of the retinoblastoma tumor suppressor gene. In addition to other, poorly understood mechanisms, Rb binds and inactivates transcription factors of the E2F type and forms transcriptional repressor complexes with histone-deacetylases (HDAC) (Zhang, H. S. *et al.,* Cell, 101, 79-89, 2000). By the phosphorylation of Rb by CDKs, bonded E2F transcription factors are released and result in transcriptional activation of genes, whose products are required for the DNA synthesis and the progression through the S-phase. In addition, the Rb-phosphorylation brings about the breakdown of the Rb-HDAC complexes by which additional genes are activated. The phosphorylation of Rb by CDKs is the equivalent of exceeding the "restriction point". For the progression through the S-phase and its completion, the activity of the CDK2/CycE and CDK2/CycA complexes is necessary, i.e., the activity of the transcription factors of the E2F type is turned off by means of phosphorylation by CDK2/CycA as soon as the cells are entered into the S-phase. After completion of the DNA replication, the CDK1 complexed with CycA or CycB controls the entry into and the passage through phases G2 and M.

According to the importance of the cell-division cycle, the passage through the cycle is strictly regulated and controlled. The enzymes necessary for the cycle progression must be activated at the correct time and subsequently turned off as soon as the corresponding phase is passed. Corresponding control points ("checkpoints") stop the progression through the cell cycle when DNA damage is detected, or when DNA replication or creation of the spindle device is not yet completed.

The activity of the CDKs is directly controlled by various mechanisms such as the synthesis and degradation of cyclins, complexation of CDKs with the corresponding cyclins, phosphorylation and dephosphorylation of regulatory threonine and tyrosine radicals, and the binding of natural inhibitory proteins. Although the amount of CDK proteins in a proliferating cell is relatively constant, the amount of the individual cyclins oscillates with the passage through the cycle. For example, the expression of CycD during the early G1 phase is stimulated by growth factors, while the expression of CycE is induced after the "restriction point" are exceeded by the activation of the transcription factors of the E2F type. The cyclins themselves are degraded by the ubiquitin-mediated proteolysis. Activating and inactivating phosphorylations regulate the activities of the CDKs. An example of activating phosphorylation are the phosphorylate CDK-activating kinases (CAKs) Thr160/161 of the CDK1, while an example of inactivating phosphorylation is found with the Wee1/Myt1 families which inactivate kinases CDK1 by phosphorylation of Thr14 and Tyr15. These inactivating phosphorylations can be destroyed in turn by cdc25 phosphatases. The regulation of the activity of the CDK/Cyc complexes by two families of natural CDK inhibitor proteins (CKls), the protein products of the p21 gene family (p21, p27, p57) and the p16 gene family (p15, p16, p18, p19) is very significant. Members of the p21 family bind to cyclin complexes of CDKs 1,2,4,6, but inhibit only the complexes that contain CDK1 or CDK2, whereas members of the p16 family are specific inhibitors of the CDK4- and CDK6 complexes.

The plane of control point regulation lies above this complex direct regulation of the CDKs' activity. Control points allow the cell to track the orderly sequence of the individual phases during the cell cycle. The most important control points are found at the transition from G1 to S and from G2 to M. The G1 control point ensures that the cell does not initiate any DNA synthesis unless it has proper nutrition, that it interacts correctly with other cells or the substrate, and that its DNA is intact. The G2/M control point ensures the complete replication of DNA and the creation of the mitotic spindle before the cell enters into mitosis. The G1 control point is activated by the gene product of the p53 tumor suppressor gene. p53 is activated after detection of changes in metabolism or the genomic integrity of the cell and can trigger either a stopping of the cell cycle progression or apoptosis. In this case, the transcriptional activation of the expression of the CDK inhibitor protein p21 by p53 plays a decisive role. A second branch of the G1 control point comprises the activation of the ATM and Chk1 kinases after DNA damage by UV light or ionizing radiation and finally the phosphorylation and the subsequent proteolytic degradation of the cdc25A phosphatase (Mailand, N. *et al., Science,* 288, 1425-1429, 2000). A result of this is the shutdown of the cell cycle since the inhibitory phosphorylation of the CDKs is not removed. After the G2/M control point is activated by damage of the DNA, both mechanisms are involved in a similar way in stopping the progression through the cell cycle.

The loss of the regulation of the cell cycle and the loss of function of the control points are characteristics of tumor cells. The CDK-Rb signal path is affected by mutations in over 90% of human tumor cells. These mutations, which finally result in inactivating phosphorylation of the Rb, include the over-expression of D- and E-cyclins by gene amplification or chromosomal translocations, inactivating mutations or deletions of CDK inhibitors of the p16 type, as well as, increased (p27) or reduced (CycD) protein degradation. The second group of genes, which are affected by mutations in tumor cells, codes for components of the control points. Thus p53, which is essential for the G1 and G2/M control points, is the most frequently mutated gene in human tumors (about 50%). In tumor cells that express p53 without mutation, it is often inactivated because of a greatly increased protein degradation. In a similar way, the genes of other proteins necessary for the function of the control points are affected by mutations, such as ATM (inactivating mutations) or cdc25 phosphatases (over-expression).

Convincing experimental data indicate that CDK2/Cyc complexes occupy a decisive position during the cell cycle progression:
(1) Both dominant-negative forms of CDK2, such as the transcriptional repression of the CDK2 expression by anti-sense oligonucleotides, produce a stopping of the cell cycle progression;
(2) The inactivation of the CycA gene in mice is lethal;
(3) The disruption of the function of the CDK2/CycA complex in cells by means of cell-permeable peptides results in tumor cell-selective apoptosis (Chen, Y. N. P. *et al*., *Proc. Natl. Acad. Sci. USA*, 96, 4325-4329, 1999);
(4) Although CDK2 as well as CyclinE knock out mice are viable, indicating that CDK2 activity can be compensated during the cell division of normal cells, fibroblasts derived from CDK2 or CyclinE knock out mice are rather resistant against oncogenic transformation, which indicates the requirement of CDK2 activity for oncogenic cell cycling (Geng, Y. et al., *Cell,* 114, 431-443, 2003; Ortega, S., et al. *Nat. Genet.,* 35, 25-31, 2003).

Cell cycle control changes play a role not only in carcinoses. The cell cycle can also be activated by a number of viruses, both by transforming viruses as well as by non-transforming viruses, making it possible to replicate viruses in the host cell. The false entry into the cell cycle of normal post-mitotic cells is associated with various neurodegenerative diseases. The mechanisms of the cell cycle regulation, their changes in diseases, and a number of approaches to develop inhibitors of the cell cycle progression, especially the CDKs, have been described in detail in several publications (Sielecki, T. M. *et al*., *J. Med. Chem*., 43, 1-18, 2000; Fry, D. W. & Garrett, M. D., *Curr. Opin. Oncol. Endo. Metab. Invest. Drugs*, 2, 40-59, 2000; Rosiania, G. R. & Chang, Y. T. , *Exp. Opin. Ther. Patents,* 10, 215-230, 2000; Meijer L. *et al., Pharmacol. Ther*., 82, 279-284, 1999; Senderowicz, A. M. & Sausville, E. A., *J*. *Natl. Cancer Inst*., 92, 376-387, 2000).

On the other hand, protein ligands and receptor tyrosine kinases, such as VEGF/VEGF-receptor (VEGF-R) system, that specifically regulate endothelial cell function, are also substantially involved in physiological as well as in disease-related angiogenesis. The VEGF-R family includes Flt1 (VEGF-R1), Flk1/KDR (VEGF-R2), and Flt4 (VEGF-R3). These receptors are recognized by members of the VEGF-related growth factors in that the ligands of Flt1 are VEGF and placenta growth factor (PIGF), whereas Flk1/KDR binds VEGF, VEGF-C and VEGF-D, and the ligands of Flt4 are VEGF-C and VEGF-D (Nicosia, *Am. J*. *Pathol*. 153, 11-16, 1998).

In pathological settings which involve processes of increased neoangiogenesis, such as tumor diseases, an increased expression level of angiogenic growth factors, such as VEGF, and of the corresponding receptors is known. Inhibition of the VEGF/VEGF-R system results in inhibiting the formation of vascular systems within tumors, thereby preventing sufficient supply of oxygen and nutritions to the tumour and leading to inhibition of tumor growth.

Most solid tumors express large amounts of VEGF, and the expression of VEGF receptors is preferentially enhanced in endothelial cells which are located near the tumor or which penetrate the tumor (Plate et al., *Cancer Res.* 53, 5822-5827, 1993). It has been shown that the inactivation of the VEGF/VEGF-R system by means of VEGF-neutralizing antibodies (Kim et al., *Nature* 362, 841-844, 1993), retroviral mediated expression of dominant-negative variants of VEGF receptors (Millauer et al., *Nature* 367, 576-579, 1994), recombinant VEGF-neutraizing variants of VEGF receptors (Goldman et al., *Proc. Natl. Acad. Sci. USA* 95, 8795-8800, 1998), or low molecular weight inhibitors of the VEGF receptor tyrosine kinases (Fong et al., *Cancer Res.* 59, 99-106, 1999; Wedge et al., *Cancer Res.* 60, 970-975, 2000; Wood et al., *Cancer Res.* 60, 2178-2189, 2000) inhibits tumor growth and tumor vascularization. Thus, inhibition of tumor-induced neovascularization represents a promising therapy option for cancerous diseases.

Compounds that selectively inhibit CDKs have been described in WO 01/44217, WO 99/24416, WO 01/44242.

Compounds that selectively inhibit the VEGF/VEGF-R systems have been described in WO 98/35958, WO 03/040102, WO 00/27819.

However, although many tumors were inhibited by interference with the VEGF/VEGF-R system, others were unaffected (Millauer et al., Cancer Res. 56, 1615-1620, 1996). Human tumors as well as experimental tumor xenografts contain a large number of immature blood vessels that have not yet recruited periendothelial cells. The fraction of immature vessels is in the range of 40% in slow growing prostate cancer and 90% in fast growing glioblastoma. A selective obliteration of immature tumor vessels was observed upon withdrawal of VEGF by means of downregulation of VEGF transgene expression in a C6 glioblastoma xenograft model. This result is due to VEGF's function as endothelial cell survival factor. A similar result was seen in human prostate cancer where the shutting off VEGF expression due to androgen-ablation therapy led to selective apoptotic death of endothelial cells in vessels lacking periendothelial cell coverage. In contrast, the fraction of vessels which resisted VEGF withdrawal showed periendothelial cell coverage (Benjamin et al., J. Clin. Invest. 103, 159-165, 1999).

It is also known that the inhibition of the VEGF/VEGR-R system by various methods only slows down the growth of most experimental tumors (Millauer et al., *Nature*, 367, 576-579, 1994; Kim et al., *Nature*, 362, 841-844, 1993; Millauer et al., *Cancer Res*., 56, 1615-1620, 1996; Goldman et al., *Proc. Natl. Acad. Sci. USA,* 95, 8795-8800, 1998; Fong et al., *Cancer Res.,* 59, 99-106, 1999; Wedge et al., *Cancer Res.,* 60, 970-975, 2000; Wood et al., *Cancer Res.,* 60, 2178-2189, 2000; Siemeister et al., *Cancer Res.,* 59, 3185-3191, 1999). Even by escalation of therapeutic doses, a plateau level of therapeutic efficacy was achieved (Kim et al., *Nature*, 362, 841-844, 1993; Wood et al., *Cancer Res*., 60, 2178-2189, 2000).

Therefore, there is a pressing need to enhance the therapeutic efficacy of these anti-angiogenesis compounds. The aim of the present invention was thus to improve the therapeutic efficacy of CDK inhibitors and VEGF/VEGF-R inhibitors by combination therapy.

It has now unexpectably been found that the combination of a CDK inhibitor from the chemical class of thiazoles (Compound A) and of a VEGFNEGF-R inhibitor from the chemical classes of phthalazines and anthranilic acid amides (Compound B) (hereinafter referred to as "inventive combination") results in a synergistical effect with respect to the inhibiton of tumor growth compared with the inhibition obtained with each agent alone.

It has now been found that a pharmaceutical combination comprising at least one CDK inhibitor (Compound A) of general formula (I): 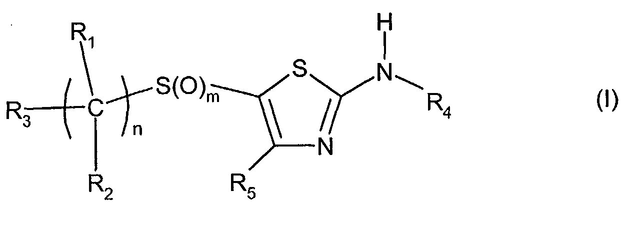 wherein:
R₁ and R₂ are independently hydrogen, fluorine or alkyl;
R₃ is aryl or heteroaryl;
R₄ is alkyl, cycloalkyl, aryl, cycloalkylalkyl, arylalkyl, heteroaryl, heteroarylalkyl, heterocycloalkyl, heterocycloalkylalkyl;
   or
CO-alkyl, CO-cycloalkyl, CO-aryl,CO-alkyl-cycloalkyl, CO-alkyl-aryl, CO-heteroaryl, CO-alkyl-heteroaryl,CO-heterocycloalkyl, CO-alkyl-heterocycloalkyl;
   or
CONH-alkyl, CONH-cycloalkyl, CONH-aryl,CONH-alkyl-cycloalkyl, CONH-alkyl-aryl, CONH-heteroaryl, CONH-alkyl-heteroaryl, CONH-heterocycloalkyl, CONH-alkyl-heterocycloalkyl;
   or
COO-alkyl, COO-cycloalkyl, COO-aryl, COO-alkyl-cycloalkyl, COO-alkyl-aryl, COO-heteroaryl, COO-alkyl-heteroaryl, COO-heterocycloalkyl, COO-alkyl-heterocycloalkyl;
   or
SO₂-cycloalkyl, SO₂-aryl, SO₂-alkyl-cycloalkyl, SO₂-alkyl-aryl, SO₂-heteroaryl, SO₂-alkyl-heteroaryl, SO₂-heterocycloalkyl, SO₂-alkyl-heterocycloalkyl;
   or
C(NCN)NH-alkyl, C(NCN)NH-cycloalkyl, C(NCN)NH-aryl, C(NCNNH)-alkyl-cycloalkyl, C(NCN) NH-alkyl-aryl, C(NCN)NH-heteroaryl, C(NCN)NH-alkyl-heteroaryl, C(NCN)NH-heterocycloalkyl, C(NCN)NH-alkyl-heterocycloalkyl;
   or
C(NNO₂)NH-alkyl, C(NNO₂)NH-cycloalkyl, C(NNO₂)NH-aryl, C(NNO₂)NH-alkyl-cycloalkyl, C(NNO₂)NH-alkyl-aryl, C(NNO₂)NH-heteroaryl, C(NNO₂)NH-alkyl-heteroaryl, C(NNO₂)NH-heterocyloalkyl, C(NNO₂)NH-alkyl-heterocycloalkyl;
   or
C(NH)NH-alkyl, C(NH)NH-cycloalkyl, C(NH)NH-aryl, C(NH)NH-alkyl-cycloalkyl, C(NH) NH-alkyl-aryl, C(NH)NH-heteroaryl, C(NH)NH-alkyl-heteroaryl, C(NH)NH-heterocycloalkyl, C(NH)NH-alkyl-heterocycloalkyl;
   or
C(NH)NHCO-alkyl, C(NH)NHCO-cycloalkyl, C(NH)NHCO-aryl, C(NH)NHCO-alkyl-cycloalkyl, C(NH)NHCO-alkyl-aryl, C(NH)NHCO-heteroaryl, C(NH)NHCO-alkyl-heteroaryl, C(NH)NHCO-heterocylcloalkyl, C(NH)NHCO-alkyl-heterocycloalkyl;
   or
C(NOR₆)NH-alkyl, C(NOR₆)NH-cycloalkyl, C(NOR₆)NH-aryl, C(NOR₆)NH-alkyl-cycloalkyl, C(NOR₆)NH-alkyl-aryl, C(NOR₆)NH-heteroaryl, C(NOR₆)NH-alkyl-heteroaryl, C(NOR₆)NH-heterocylcoalkyl, C(NOR₆)NH-alkyl-heterocycloalkyl;
R₅ is hydrogen or alkyl;
R₆ is hydrogen, alkyl, cycloalkyl, aryl, cycloalkylakyl, arylalkyl, heteroaryl, heteroarylalkyl, heterocycloalkyl or heterocycloalkylalkyl;
m is an integer of 0 to 2; and
n is an integer of 1 to 3;
or a pharmaceutically acceptable salt, enantiomer, diastereomer, isomer or tautomer thereof; the meaning or definition of each term being the same as described in WO 01/44217;
and at least one VEGF-R inhibitor (Compound B) of general formula (II): 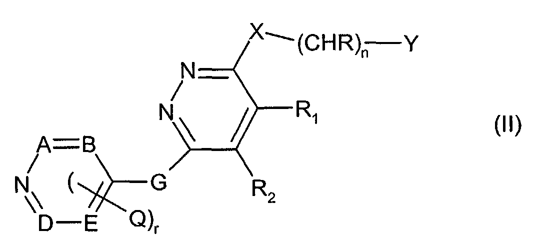 wherein:
R₁ and R₂ (i) are lower alkyl or (ii) together form a bridge of subformula I* 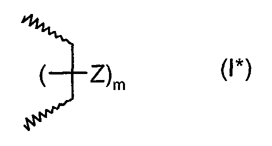 the binding being achieved via the two terminal carbon atoms, or (iii) R₁ and R₂ together form a bridge of subformula I**

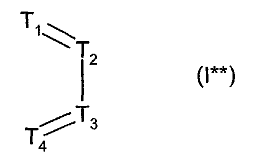

wherein one or two of the ring members T₁, T₂, T₃ and T₄ are nitrogen, and the others are in each case CH, and the binding is achieved via T₁ and T₄;
A, B, D, and E are in each case CH;
G is lower alkylene, especially methylene;
Q is methyl, which is bound to A, to D, or to A and D;
R is H or lower alkyl;
X is imino;
Y is phenyl, which is unsubstituted or substituted by one or two substituents independently of one another from the group comprising amino, lower alkanoylamino, halogen, lower alkyl, halogen-lower alkyl, hydroxy, lower alkoxy, phenyl-lower alkoxy, and cyano, or is pyridyl;
Z is amino, N-lower alkylamino, hydroxy-lower alkylamino, phenyl-lower alkylamino, N,N-dilower alkylamino, n-phenyl-lower alkyl-N-lower alkylamino, N, N-di-lower alkylphenylamino, lower alkanoylamino; or a substituent from the group comprising benzoylamino or phenyllower alkoxycarbonylamino, wherein the phenyl radical in each case is unsubstituted or especially substituted by nitro or amino, or by halogen, amino, N-lower alkylamino, N,N-dilower alkylamino, hydroxy, cyano, carboxy, lower alkoxycarbonyl, lower alkanoyl or carbamoyl; or is halogen; and,
r is 0 to 2;
n is 0 or 1;
m is 0 or 1;
the bonds characterized by a wavy line are in each case a double bond or in each case a single bond;
or a pharmaceutically acceptable salt, enantiomer, diastereomer, isomer or tautomer thereof; the meaning or definition of each term being the same as described in WO 98/35958;
or of general formula (IIa): 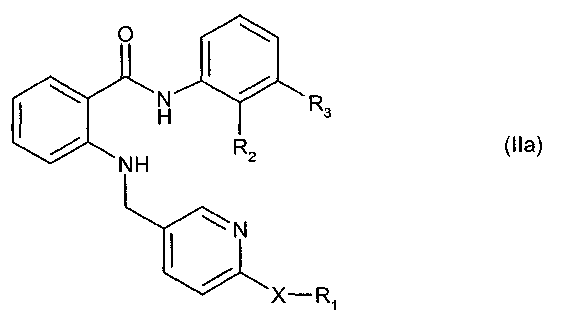 wherein:
R₁ is H or lower alkyl;
R₂ is H or lower alkyl;
R₃ is perfluoro lower alkyl; and
X is O or S,
or an N-oxide or a tautomer thereof,
or a pharmaceutically acceptable salt, enantiomer, diastereomer, isomer or tautomer thereof; the meaning or definition of each term being the same as described in WO 03/040102;
or of general formula (IIb): 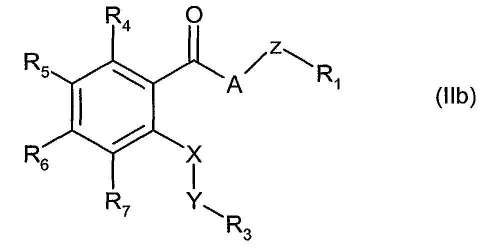 wherein:
A is NR₂
Z is NH, NR₁₀, N(R₁₀)-(CH₂)_{q} or the group 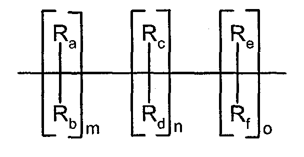 or A, Z and R₁ together form one of the following groups: 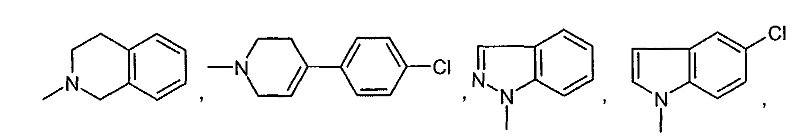 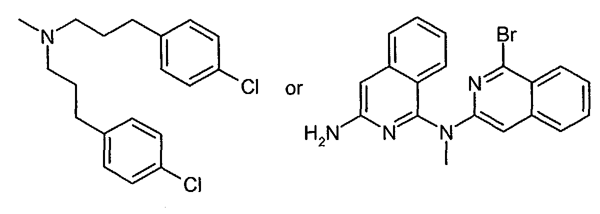
m, n and o are 0 - 3;
q is 1- 6;
Rₐ, R_{b}, R_{c}, R_{d}, Rₑ, R_{f}, independently of one another, are hydrogen or methyl or the group NR₁₀;
X is NR₉;
Y is CH₂;
R₁ is phenyl, pyridyl, 5-chloro-2,3-dihyroindenyl, 2,3-dihydroindenyl, thienyl, 6-fluoro-1 H-indol-3-yl, naphthyl, 1,2,3,4-tetrahydronaphthyl, benzo-1,2,5-oxadiazole or 6,7-dimethoxy-1,2,3,4-tetrahydro-2-naphthal or is phenyl or pyridyl that is substituted in one or more places with C₁-C₄ alkyl, C₁-C₄ alkoxy, hydroxy, halogen, or trifluoromethyl or is one of the following groups: 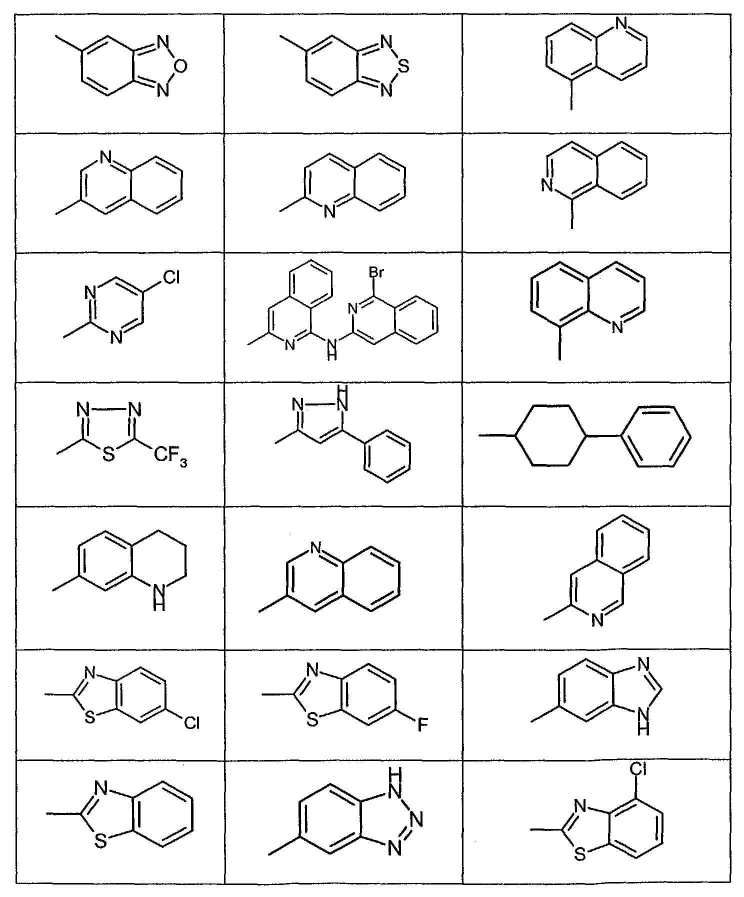 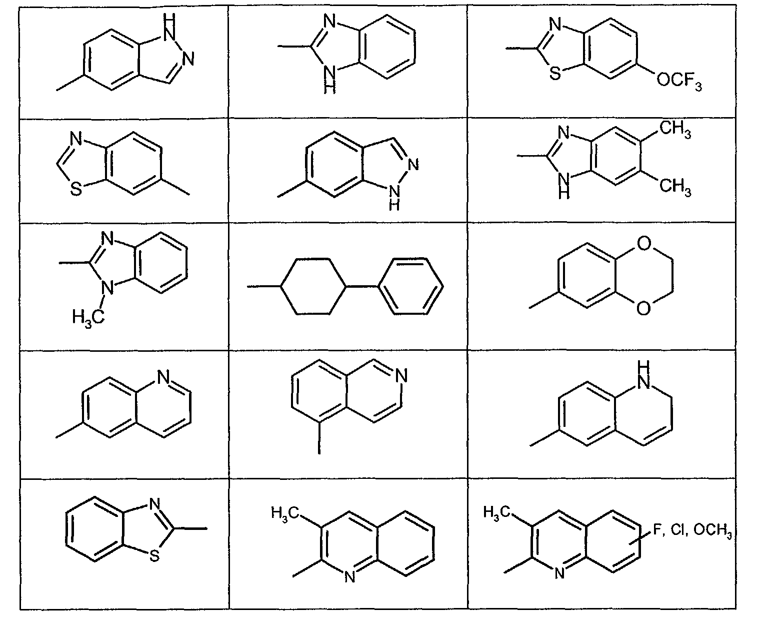
whereby phenyl or substituted phenyl or naphthyl is not directly bonded to the NR₂ group in the meaning of A;
R₂ is H or CH₃;
R₃ is pyridyl or phenyl, pyridyl or 1,2,3,4-tetrahydronaphthyl that is substituted in one or more places with hydroxy, halogen, methyl or methoxy, or is one of the following groups: 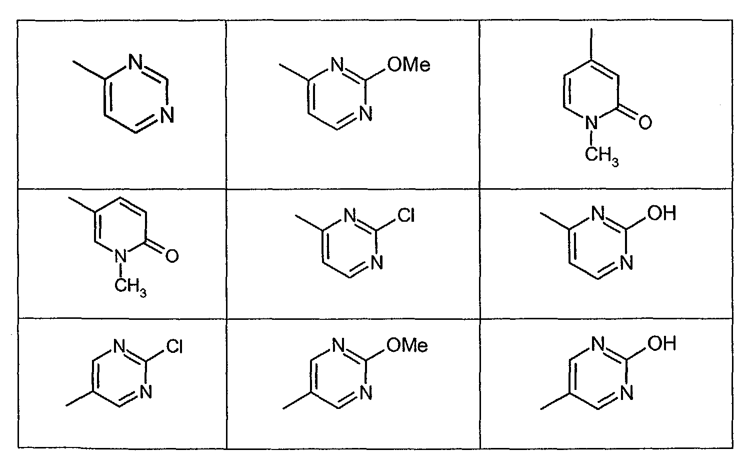 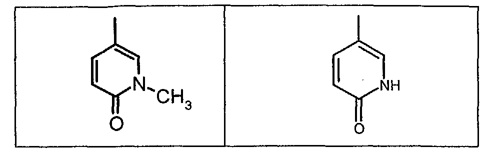
R₅ and R₆, independently of one another, are hydrogen, halogen, methyl, methoxy or trifluoromethyl;
R₄ and R₇, independently of one another, are hydrogen or halogen;
R₉ is H or C₁-C₆ alkyl;
R₁₀ is H or methyl;
or a pharmaceutically acceptable salt, enantiomer, diastereomer, isomer or tautomer thereof, will overcome the known disadvantages.

The meaning or definition of terms used in formula (IIb) are the same as described in WO 00/27819;

Compounds described in WO 01/44217, WO 99/24416 and WO 01/44242 (Compound A) and compounds described in WO 98/35958, WO 03/040102, and WO 00/27819 (Compound B) are herein incorporated by reference.

Preferred compounds for Compound A for use in the present inventive combination include any of the CDK inhibitors of Examples 1 to 735 disclosed in WO 01/44217, Examples 1 to 637 disclosed in WO 99/24416, and Examples 1 to 19 disclosed in WO 01/44242, or any other pharmaceutically acceptable salt, enantiomer, diastereomer, isomer or tautomer thereof.

Compound A is more preferably a compound of general formula (I) wherein:
R₁ and R₂ are independently hydrogen, fluorine or alkyl;
R₃ is 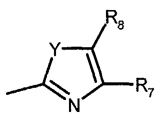 wherein Y is O, S or NR₉;
R₄ is alkyl, cycloalkyl, aryl, cycloalkylalkyl, arylalkyl, heteroaryl, heteroarylalkyl, heterocycloalkyl, heterocycloalkylalkyl;
   or
CO-alkyl, CO-cycloalkyl, CO-aryl,CO-alkyl-cycloalkyl, CO-alkyl-aryl, CO-heteroaryl,CO-alkyl-heteroaryl, CO-heterocycloalkyl, CO-alkyl-heterocycloalkyl;
   or
CONH-alkyl, CONH-cycloalkyl, CONH-aryl, CONH-alkyl-cycloalkyl, CONH-alkyl-aryl, CONH-heteroaryl, CONH-alkyl-heteroaryl,CONH-heterocycloalkyl, CONH-alkyl-heterocycloalkyl;
   or
COO-alkyl, COO-cycloalkyl, COO-aryl, COO-alkyl-cycloalkyl, COO-alkyl-aryl, COO-heteroaryl, COO-alkyl-heteroaryl, COO-heterocycloalkyl, COO-alkyl-heterocycloalkyl;
   or
SO₂-cycloalkyl, SO₂-aryl, SO₂-alkyl-cycloalkyl, SO₂-alkyl-aryl, SO₂-heteroaryl, SO₂-alkyl-heteroaryl, SO₂-heterocycloalkyl, SO₂-alkyl-heterocycloalkyl;
   or
C(NCN)NH-alkyl, C(NCN)NH-cycloalkyl, C(NCN)NH-aryl, C(NCNNH)-alkyl-cycloalkyl, C(NCN)NH-alkyl-aryl, C(NCN)NH-heteroaryl, C(NCN)NH-alkyl-heteroaryl, C(NCN)NH-heterocycloalkyl, C(NCN)NH-alkyl-heterocycloalkyl;
   or
C(NNO₂)NH-alkyl, C(NNO₂)NH-cycloalkyl, C(NNO₂)NH-aryl, C(NNO₂)NH-alkyl-cycloalkyl, C(NNO₂)NH-alkyl-aryl, C(NNO₂)NH-heteroaryl, C(NNO₂)NH-alkyl-heteroaryl, C(NNO₂)NH-heterocyloalkyl, C(NNO₂)NH-alkyl-heterocycloalkyl;
   or
C(NH)NH-alkyl, C(NH)NH-cycloalkyl, C(NH)NH-aryl, C(NH)NH-alkyl-cycloalkyl, C(NH)NH-alkyl-aryl, C(NH)NH-heteroaryl, C(NH)NH-alkyl-heteroaryl, C(NH)NH-heterocycloalkyl, C(NH)NH-alkyl-heterocycloalkyl;
   or
C(NH)NHCO-alkyl, C(NH)NHCO-cycloalkyl, C(NH)NHCO-aryl, C(NH)NHCO-alkyl-cycloalkyl, C(NH)NHCO-alkyl-aryl, C(NH)NHCO-heteroaryl, C(NH)NHCO-alkyl-heteroaryl, C(NH)NHCO-heterocylcloalkyl, C(NH)NHCO-alkyl-heterocycloalkyl;
   or
C(NOR₆)NH-alkyl, C(NOR₆)NH-cycloalkyl, C(NOR₆)NH-aryl, C(NOR₆)NH-alkyl-cycloalkyl, C(NOR₆)NH-alkyl-aryl, C(NOR₆)NH-heteroaryl, C(NOR₆)NH-alkyl-heteroaryl, C(NOR₆) NH-heterocylcoalkyl, C(NOR₆)NH-alkyl-heterocycloalkyl; R₅ is hydrogen;
R₆ is hydrogen, alkyl, cycloalkyl, aryl, cycloalkylakyl, arylalkyl, heteroaryl, heteroarylalkyl, heterocycloalkyl or heterocycloalkylalkyl;
R₇ and R₈ are independently hydrogen, alkyl, cycloalkyl, aryl, alkylcycloalkyl, alkylaryl, heteroaryl, alkylheteroaryl, heterocycloalkyl, alkylheterocycloalkyl or halogen;
R₉ is H or alkyl;
m is the integer 0; and
n is the integer 1.

More preferred for Compound A are compounds of general formula (I) wherein :
R₁ is H;
R₂ is H, F or alkyl;
R₃ is a substituted oxazole having the configuration : 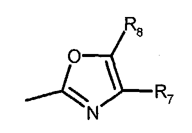
R₄ is CO-alkyl, CO-alkyl-aryl, CO-cycloalkyl, CO-alkyl-heteroaryl, CO-alkyl-heteroalkyl, CO-heterocycloalkyl, CO-alkyl-heterocycloalkyl, aryl, arylalkyl, heteroaryl, heteroarylalkyl;
R₅ is H;
R₇ is H;
R₈ is an alkyl group, such as tert-butyl;
m is the integer 0; and
n is the integer 1.

Most preferred for Compound A are compounds of general formula (I) wherein:
R₁ is H;
R₂ is H;
R₃ is a substituted oxazole having the configuration : 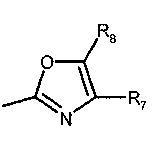
R₄ is CO-heterocycloalkyl or CO-cycloalkyl;
R₅ is hydrogen;
R₇ is hydrogen;
R₈ is tert-butyl;
m is the integer 0; and
n is the integer 1;
or more particularly the following compounds:
N-[5-[[[5-(1,1-dimethylethyl)-2-oxazolyl] methyl]thio]-2-thiazolyl]-4-piperidinecarboxamide;
(±)-N-[5-[[[5-(1,1-dimethylethyl)-2-oxazolyl] methyl]thio]-2-thiazolyl]-3-piperidinecarboxamide;
(±)-1-(2,3-dihydroxypropyl)-N-[5-[[[5-(1,1-dimethylethyl)-2-oxazolyl]-methyl]thio]-2-thiazolyl]-4-piperidinecarboxamide;
N-[5-[[[5-(1,1-dimethylethyl)-2-oxazolyl] methyl]thio]-2-thiazolyl]-1-(1-methylethyl)-4-piperidinecarboxamide;
1-cyclopropyl-N-[5-[[[5-(1,1-dimethylethyl)-2-oxazolyl]methyl]thio]-2-thiazolyl]-4-piperidinecarboxamide;
N-[5-[[[5-(1,1-dimethylethyl)-2-oxazolyl] methyl]thio]-2-thiazolyl]-1-(2-hydroxyethyl)-4-piperidinecarboxamide;
(R)-N-[5-[[[5-(1,1-dimethylethyl)-2-oxazolyl]methyl]thio]-2-thiazolyl]-3-piperidinecarboxamide;
(S)-N-[5-[[[5-(1,1-dimethylethyl)-2-oxazolyl] methyl]thio]-2-thiazolyl]-3-piperidinecarboxamide;
cis-4-amino-N-[5-[[[5-(1,1-dimethylethyl)-2-oxazolyl] methyl]thio]-2-thiazolyl] cyclohexylcarboxamide;
trans-4-amino-N-[5-[[[5-(1,1-dimethylethyl)-2-oxazolyl] methyl]thio]-2-thiazolyl] cyclohexylcarboxamide;
N-[5-[1[5-(1,1-Dimethylethyl)-2-oxazolyllmethyllthiol-2-thiazolyll-4-piperidinecarboxamide.

Preferred compounds for Compound B for use in the present inventive combination include any of the VEGF-R inhibitors of Examples 1 to 82 disclosed in WO 98/35958, Examples 1 to 6 disclosed in WO 03/040102, and Examples 1.0 to 11.0 disclosed in WO 00/27819, or any other pharmaceutically acceptable salt, enantiomer, diastereomer, isomer or tautomer thereof.

Compound B is more preferably a compound of general formula (II) wherein:
R₁ and R₂ together form a bridge in subformula I*, the binding being achieved via the two terminal carbon atoms;
A, B, D, and E are in each case CH;
G is methylene;
R is H;
X is imino;
Y is phenyl, which is substituted or unsubstituted by one or two substituents independently of one another from the group comprising amino, lower alkanoylamino, halogen, lower alkyl, halogen-lower alkyl, hydroxy, lower alkoxy, phenyl-lower alkoxy, and cyano;
r is 0;
n is 0 or 1;
m is 0; and
the bonds characterized by a wavy line are double bonds;
or a compound of general formula (IIa) wherein:
R₁ is H or methyl;
R₂ is H or methyl;
R₃ is trifluoromethyl; and
X is O;
or a compound of general formula (IIb) wherein:
Z is the group 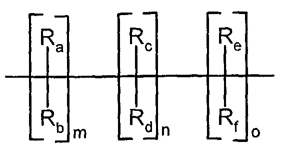 and m, n, and o are 0;
or A, Z and R₁ together form one of the following groups: 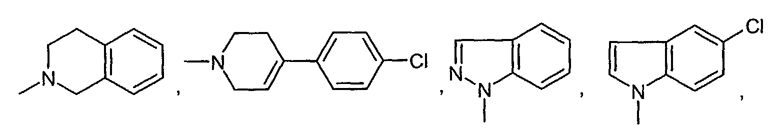 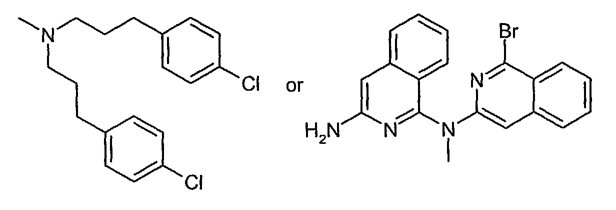
R₉ is H; and
R₄, R₅, R₆, and R₇, independently of one another are H or halogen;

Most preferred for Compound B are the following three compounds: 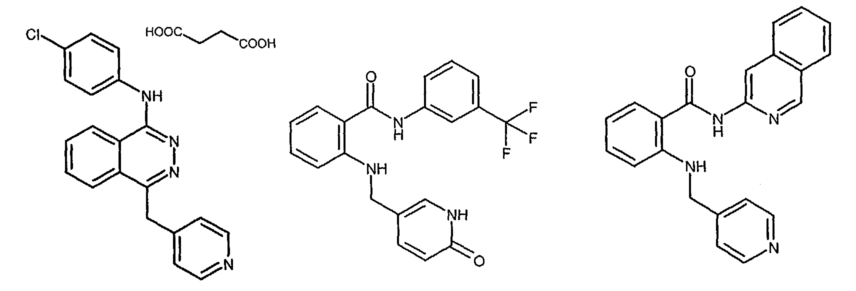 or a pharmaceutically acceptable salt, enantiomer, diastereomer, isomer or tautomer thereof.

Compounds A of the inventive combination can be prepared as already described in WO 01/44217, WO 99/24416, and WO 01/44242.

Compounds B of the inventive combination can be prepared as already described in WO 98/35958, WO 03/040102, and WO 00/27819.

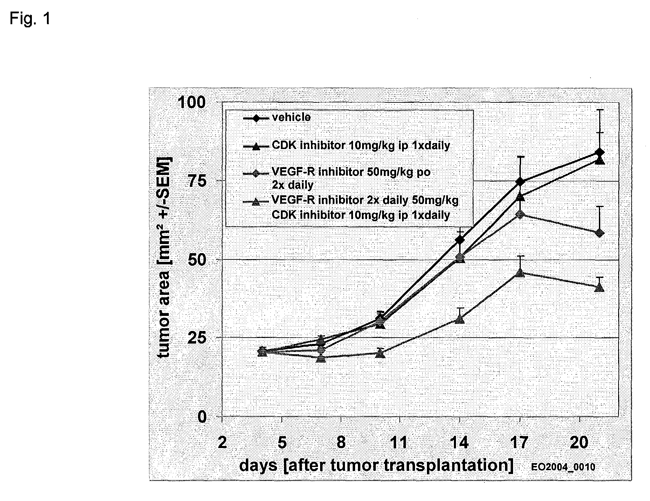

The inventive combination comprising at least one compound of general formula (I) and at least one compound of general formula (II), (IIa) and/or (IIb) has been found to have superior pharmaceutical activity in the treatment of cancer and tumors as compared to each compound alone. An example of this superior activity is found in Example 1, where the combination of compounds of general formula (I) and general formula (II) has been shown to rapidly reduce tumor growth (see Figure 1).

By the term "combination" is meant any pharmaceutical combination in which Compound A and Compound B are administered in a single dose unit, for example a single tablet or a capsule containing a fixed ratio of the two active ingredients (i.e. Compound A and Compound B), as well as combination therapy in which Compound A and Compound B are administered in separate dosages, that is to say, administration of each agent simultaneously or sequentially.

The inventive combination may be used on its own, or preferably as a pharmaceutical composition in which the active ingredients are in admixture with a pharmaceutically acceptable adjuvant, diluent or carrier. Therefore, according to a further aspect of the invention there is provided a pharmaceutical composition comprising the inventive combination as hereinbefore defined in association with a pharmaceutically acceptable adjuvant, diluent or carrier.

An example is a pharmaceutical composition in a form appropriate for enteral or parental administration. Enteral administration includes nasal, buccal, rectal or especially oral administration, while parenteral administration includes intravenous, intramuscular or subcutaneous administration.

The route of administration and the dosage administered is not limited and can, of course, vary with the compounds employed, the treatment desired, as well as the species, gender, age, weight, individual condition, severity of the disease, individual pharmacokinetic data and other conditions, as appropriate. The amount of active ingredients may be generally about 0.0001 to 100 mg/kg a day. Preferably a unit dosage form contains about 0.001 to 1000 mg of active ingredients.

The pharmaceutical composition can be prepared in a manner well-known to a person skilled in the art, for example, by means of conventional mixing, granulating, coating, dissolving or lyophilizing processes.

Preferred are pharmaceutical compositions for oral administration which can be obtained, for example, by combining the active ingredients with one or more solid carriers, if desired granulating a resulting mixture, and processing the mixture of granules, if desired or necessary, by the inclusion of additional excipients, to form tablets or tablet cores.

Examples of suitable adjuvants, diluents and carriers are well known to a person skilled in the art and include sugars such as lactose, saccharose, mannitol or sorbitol, cellulose preparations, and/or calcium phosphates, such as tricalcium phosphate or calcium hydrogen phosphate, and binders, such as starches, for example corn, wheat, rice or potato starch, methylcellulose, hydroxypropyl methylcellulose, sodium carboxymethyl-cellulose, and/or polyvinylpyrrolidone, and/or, if desired, disintegrators, such as the above-mentioned starches, and additionally carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

More specifically, for preparing tablets the following carriers well-known in the art may be used: excipients such as lactose, glucose, starch, calcium carbonate, kaoline, crystalline cellulose and silicic acid; binders such as water, ethanol, propanol, simple syrup, glucose solution, starch solution, gelatin solution, carboxymethyl cellulose, shellac, methyl cellulose and polyvinyl pyrrolidone; disintegrators such as dried starch, sodium alginate, powdered agar, calcium carmelose, starch and lactose; disintegration retarders such as sucrose, cocoa butter and hydrogenated oil; absorption promoters such as quaternary ammonium base and sodium lauryl sulfate; moisturizing agents such as glycerin and starch; adsorbents such as starch, lactose, kaoline, bentonite, colloidal silicic acid; and glidants such as talc, stearates and polyethylene glycol. The tablet may be coated with a common coating such as a sugar coating, gelatin coating, enteric coating, film coating, double layer coating, and micro layer coating.

Capsules may be prepared by blending the active ingredients with a variety of the above carriers in a usual manner and filling the resulting blend into, for example, a hard or soft gelatin capsule.

Tablet cores can be provided with suitable, optionally enteric, coatings through the use of, *inter alia*, concentrated sugar solutions, which include gum arabic, talc, polyvinylpyrrolidone, polyethzlene glycol and/or titanium dioxide, or coating solutions in suitable organic solvents or solvent mixture, or for the preparation of enteric coatings, solutions of suitable cellulose preparations, such as acetly cellulose phthalate or hydroxypropylmethylcellulose phthalate. Dyes or pigments may be added to the tablets or tablet coatings, for example for identification purposes or to indicate different doses of the active ingredients.

Pharmaceutical compostions for oral administration also include hard capsules consisting of gelatin or soft, sealed capsules consisting of gelatin and a plasticizer, such as glycerol or sorbitol. The hard capsules may contain the active ingredients in the form of granules, for example in admixture with fillers, such as corn starch, binders, and/or glidants, such as talc or magnesium stearate, and optionally stabilizers. In soft capsules, the active ingredients are preferably dissolved or suspended in suitable liquid excipients, such as fatty oils, paraffin oil or liquid polyethylene glycols or fatty acid esters of ethylene or propylene glycol, to which stabilizers and detergents, such as a polyoxyethylene sorbitan fatty acid ester, may also be added.

For the preparation of pills, the following carriers well-known in the art may be used: excipients such as crystalline cellulose, lactose, starch, hydrogenated vegetable oil, kaoline and talc; binders such as powdered acacia, powdered tragacanth gum and gelatin; and disintegrators such as calcium carmelose and agar.

Other oral dosage forms are, for example, syrups prepared in customary manner which comprise the active ingredients in suspended form and in a concentration of about 5% to 20%, preferably about 10%, or in similar concentration that provides a suitable single dose, for example, a 5 or 10 ml dose. Also suitable are, for example, powdered or liquid concentrates for the preparation of shakes in for example milk. Such concentrates may also be packaged in single-dose units.

Solutions, suspensions and dispersions of the active ingredients may be sterilized and/or may comprise excipients such as preservatives, stabilizers, wetting agents and/or emulsifiers, solubilizers, salts for regulating osmotic pressure and/or buffers and are prepared in a manner known per se, that is, by means of conventional dissolving and lyophilizing processes. The said solutions or suspensions may additionally comprise visosity-increasing agents, typically sodium carboxymethylcellulose, carboxymethylcellulose, dextran, polyvinylpyrrolidone, or gelatins, and/or solubilizers such as Tween 80 [polyoxyethylene(20)sorbitan mono-oleate; trademark of ICI Americas, Inc. USA].

Suspensions in oil can comprise as the oil component the vegetable, synthetic, or semi-synthetic oils customary for injection purposes. Especially mentioned are liquid fatty acid esters that contain as the acid component a long-chained fatty acid having from 8 to 22, especially from 12 to 22, carbon atoms, such as lauric acid, tripdecylic acid, myristic acid, pentadecylic acid, palmitic acid, margaric acid, stearic acid, arachidic acid, behenic acid or corresponding unsaturated acids such as oleaic acid, elaidic acid, erucic acid, brassidic acid or linoleic acid, if desired with the addition of anti-oxidants, such as vitamine E, β-carotene or 3,5-di-tert-butyl-4-hydroxytoluene. The alcohol component of these fatty acid esters has a maximum of 6 carbon atoms and is a monovalent or polyvalent, for example a mono-, di- or trivalent alcohol such as methanol, ethanol, propanol, butanol or pentanol or the isomers thereof, but especially glycol and glycerol. As fatty acid esters, therefore, the following are mentioned: ethyl oleate, isopropyl myristate, isopropyl palmitate, "Labrafil M 2375" (polyoxyethylene glycerol trioleate from Gattefossé, Paris), "Labrafil M 1944 CS" (unsaturated polyglycolized glycerides prepared by alcoholysis of apricot kernel oil and consisting of glycerides and polyethylene glycol ester from Gattefossé, France), "Labrasol" (saturated polyglycolized glycerides prepared by alcoholysis of TCM and consistitn of glycerides and polyethylene glycol ester from Gattefossé, France), and/or "Miglyol 812" (triglyceride of saturated fatty acids of chain length C₈ to C₁₂ from Hüls AG, Germany), but especially vegetable oils such as cottonseed oil, almond oil, olive oil, castor oil, sesame oil, soybean oil and more expecially groundnut oil.

The manufacture of injectable preparations, the filing into ampoules or vials, and the sealing of the containers is usually carried out under sterile conditions.

Pharmaceutical compositions suitable for rectal administration include, suppositories that consist of a combination of the active ingredients and a suppository base such as natural, semi-synthetic or synthetic triglycerides, cocoa butter, paraffin hydrocarbons, polyethylene glycols, higher alcohol esters or higher alkanols.

For parenteral administration, aqueous solutions of the active ingredients in water-soluble form, for example a water-soluble salt, or aqueous injection suspensions that contain viscosity-increasing substances such as sodium carboxy-methylcellulose, sorbitol and/or dextran, and, if desired, stabilizers, are especially suitable. The active ingredients optionally together with excipients, can also be in the form of a lyophilizate and can be made into a solution before parenteral administration by the addition of suitable solvents. Solutions used for parenteral administration can also be employed as infusion solutions.

Furthermore, the pharmaceutical composition may contain coloring agents, preservatives, perfumes, flavors, sweeteners and/or other drugs. Preferred preservatives are, for example, antioxidants, such as ascorbic acid, or microbicides, such as sorbic acid or benzoic acid.

In a further aspect, the invention relates to a kit comprising at least one or more unit doses of a compound of general formula (I) and at least one or more unit doses of a compound of general formula (II), (IIa) or (IIb). Such kits can, for example, be in the form of blister packs containing each medicament in separate unit doses.

Unit dose forms are, for example, coated and uncoated tablets, ampoules, vials, suppositories, capsules, ointments, creams, pastes, foams, tinctures, lip-sticks, drops, sprays, dispersions, etc.

Another aspect of the invention provides a method of treatment or prophylaxis of cancer and tumor diseases in a person or animal suffering from or susceptible to such a disease, which method comprises administering to the person or animal a therapeutically effective amount of the inventive combination either simultaneously, separately, or sequentially.

Another aspect of the invention provides the use of the inventive combination described above, in the manufacture of a medicament for the treatment or prophylaxis of cancer and tumor diseases.

In addition to the treatment of cancer and tumour diseases, which include, *inter alia,* solid tumors and leukemia, the inventive combination can also be used for the treatment of auto-immune diseases such as psoriasis, alopecia, multiple sclerosis, chemotherapy-induced alopecia and mucositis; cardiovascular diseases such as stenoses, arterioscleroses and restenoses; infectious diseases caused by unicellular parasites, such as trypanosoma, toxoplasma or plasmodium, or produced by fungi; nephrological diseases, such as glomerulonephritis; chronic neurodegenerative diseases, such as Huntington's disease, amyotropic lateral sclerosis, Parkinson's disease, AIDS dementia and Alzheimer's disease; acute neurodegenerative diseases, such as ischemias of the brain and neurotraumas; viral infections, such as cytomegalic infections, herpes, Hepatitis B and C, and HIV diseases.

In a preferred aspect of the invention, the inventive combination is suitable for administration to a warm-blooded animal, especially a person or commercially useful mammal suffering from a disease such as psoriasis or especially from a neoplastic disease, and comprises a pharmaceutically effective quantity of a compound of general formula (I) in combination with a compound of general formula (II), (IIa), or (IIb), together with at least one pharmaceutically acceptable carrier.

In another preferred aspect of the invention, the inventive combination can be used for the prophylactic or especially therapeutic management of neoplastic and other proliferative diseases of a warm-blooded animal, especially a person or a commercially useful mammal requiring such treatment, which is especially suffering from such a disease.

According to a further aspect of the invention there is thus provided a process or a method for the treatment of one of the pathological conditions mentioned hereinabove, especially a disease which responds to an inhibition of the CDK and VEGF-R, more especially a corresponding neoplastic disease or also psoriasis. The inventive combination can be administered as such or especially in the form of a pharmaceutical composition, prophylactically or therapeutically, preferably in an amount effective against the said diseases, to a warm-blooded animal, such a person requiring such treatment. In the case of an individual having a bodyweight of about 70 kg, the daily dose administered is from approximately 0.01 g to approximately 5 g, preferably from approximately 0.02 g to approximately 2 g, of active ingredients of the present invention.

In a further aspect the invention especially provides the use of the inventive combination, as such or in the form of a pharmaceutical composition with at least one pharmaceutically acceptable carrier for the therapeutic and also prophylactic management of one or more of the diseases mentioned hereinabove, especially a neoplastic disease or psoriasis, more especially a disease which responds to an inhibition of CDK and VEGF-R.

In a further aspect the invention especially provides the use of the inventive combination, as such or in the form of a pharmaceutical composition with at least one pharmaceutically acceptable carrier, diluent or excipient for the therapeutic and also prophylactic management of one or more of the diseases mentioned hereinabove, preferably a disease which responds to an inhibition of CDK and VEGF-R, especially a neoplastic disease or also psoriasis.

The invention also especially provides the use of the inventive combination for the preparation of a pharmaceutical formulation for the therapeutic and also prophylactic management of one or more of the diseases mentioned hereinabove, especially a neoplastic disease or also psoriasis, more especially a disease which responds to an inhibition of CDK and VEGF-R.

The inventive combination also has differentiation-inducing effects and can thus be used as a therapeutic and/or improving combined agent to a variety of other diseases such as malignant tumors, autoimmune diseases, dermatologic diseases and parasitism.

As used herein, a "malignant tumor" includes hematologic malignancy such as acute leukemia, malignant lymphoma, microltiple myeloma and macroglobulinemia as well as solid tumors such as colon cancer, cerebral tumor, head and neck tumor, breast carcinoma, pulmonary cancer, esophageal cancer, gastric cancer, hepatic cancer, gallbladder cancer, bile duct cancer, pancreatic cancer, nesidioblastoma, renal cell carcinoma, adrenocortical cancer, urinary bladder carcinoma, prostatic cancer, testicular tumor, ovarian carcinoma, uterine cancer, chorionic carcinoma, thyroid cancer, malignant carcinoid tumor, skin cancer, malignant melanoma, osteogenic sarcoma, soft tissue sarcoma, neuroblastoma, Wilms tumor and retinoblastoma.

An autoimmune disease includes rheumatism, such as rheumatoid arthritis, diabetes, systemic lupus erythematodes, human autoimmicrone lymphocytotic lymphadenopathy, immicro noblastic lymphadenopathy, Crohn's disease and ulcerative colitis.

A dermatologic disease includes psoriasis, acne, eczema and atopic dermatitis.

Parasitism includes diseases such as malaria caused through vermination.

In a further aspect of the invention, the inventive combination can be used for the treatment haemeangioma, angiofribroma, diseases of the eyes, such as diabetic retinopathie, neovascular glaucoma, diseases of the kidney, such as glomerulonephritis, diabetic nephropatic diseases, malignant nephrosclerosis, thrombotic microangiopatic syndrome, disposes of transplants and glomerulopathy, fibrotic diseases, such as liver cirrhosis, mesangialic cell proliferative diseases and artheriosclerosis, injury of the nervous tissues, for inhibition of reocclusion of vascular systems after balloon catheter treatment, for artificial limbs, or after insert of mechanically devices for keeping open of vasculature, such as stents.

For the treatment of injury of the nervous tissues a rapid production of scars at the place of injury can be prevented. Thus, production of scars will be prevented before the axones can re-establish. Therefore a re-construction of nerves is possible. Therefore, a further aspect of the invention provides for the use of the inventive combination in the suppression of ascites production and oedema within patients.

However, uses for the inventive combination are not limited to these specific examples.

The invention is illustrated by the experimental data given below.

### Biological Examples

### Example 1

MaTu (EPO-GmbH, Berlin) estrogen-independent human mammary carcinoma cells, obtained from cell culture, were implanted s.c. in the inguinal region of femal NMRI nude mice. Treatment was started when the tumors were approximately 20 mm² in size. The groups were closed when the tumors within a treatment group reached a size of approximately 100 mm².

Experimental groups included the following:
1) Control vehicle
2) a CDK inhibitor (10 mg/kg, i.p. once daily, d4-d21)
3) a VEGF-R inhibitor (50 mg/kg, p.o. twice daily, d4-d21)
4) a CDK inhibitor (10 mg/kg, i.p. once daily, d4-d21) in combination with a VEGF-R inhibitor (50 mg/kg, p.o. twice daily, d4-d21)
CDK inhibitor = N-[5-[[[5-(1,1-dimethylethyl)-2-oxazolyl] methyl]thio]-2-thiazolyl]-4-piperidinecarboxamide
VEGF-R inhibitor = (4-Chlorophenyl)[4-(4-pyridylmethyl)phthalazin-1-yl]ammonium hydrogen succinate

Tumor area was determined by caliper measurement twice weekly.
The example was designed to determine the initial response of a human estrogen-independent mammary carcinoma xenograft model to treatment with a specific CDK inhibitor, to treatment with a specific VEGF-R inhibitor, and to treatment with a combination of a CDK inhibitor together with a VEGF-R inhibitor. The tumor growth inhibiting effect of the compounds was investigated in the MaTu human estrogen-independent mammary carcinoma model, xenografted in NMRI nude mice.

The results (**Figure 1**) of the study demonstrate that the selective CDK inhibitor was ineffective in inhibition of the growth of the MaTu xenograft model in monotherapy at a dosing of 10 mg/ml i.p. once daily. The selective VEGF-R inhibitor given at a dosing of 50 mg/kg p.o. twice daily showed during the time course of the study initially an inhibition of tumor growth. Upon reaching a larger tupor size of approx. 60 mm², inhibition of VEGF-induced neoangiogenesis appeared to result in inhibition of tumor growth. The combination of both compounds at a dosing of 10 mg/kg i.p. once daily for the CDK inhibitor and 50 mg/kg p.o. twice daily for the VEGF-R inhibitor showed a clear, synergistical or substantially greater, inhibition of tumor growth in comparison to monotherapy and the control group observed. The reduction of the tumor size with combination therapy was statistically significant (p<0.05) with respect to the control group.

The results show that a combination therapy using a CDK inhibitor and VEGF-R inhibitor is substantially superior in the efficacy of tumor growth inhibition as compared to monotherapy with the each of the separate compounds. The observation of a more than additive inhibition of tumor growth upon combination treatment with the compounds, which achieved no or only delayed inhibition of tumor growth upon monotherapy, represents an unexpected result of the study.

This data shows clear superiority of the combination over the single agents if applied alone.

### Description of the Figures

**Figure 1** shows the efficacy in the human MaTu estrogen-independent mammary carcinoma xenograft model. MaTu tumor cells were implanted s.c. into nude mice on day 0. Treatment was started on day 4 when tumors had reached a size of approximately 20 mm². A CDK inhibitor and VEGF-R inhibitor were given in the doses and schedules as indicated. The time course of tumor growth is depicted.

## Claims

1. A pharmaceutical combination comprising a CDK inhibitor (Compound A) of general formula (I): 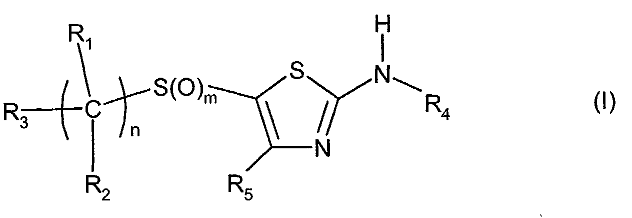 wherein:
R₁ and R₂ are independently hydrogen, fluorine or alkyl;
R₃ is aryl or heteroaryl;
R₄ is alkyl, cycloalkyl, aryl, cycloalkylalkyl, arylalkyl, heteroaryl, heteroarylalkyl, heterocycloalkyl, heterocycloalkylalkyl;
or
CO-alkyl, CO-cycloalkyl, CO-aryl,CO-alkyl-cycloalkyl, CO-alkyl-aryl, CO-heteroaryl, CO-alkyl-heteroaryl,CO-heterocycloalkyl, CO-alkyl-heterocycloalkyl;
or
CONH-alkyl, CONH-cycloalkyl, CONH-aryl,CONH-alkyl-cycloalkyl, CONH-alkyl-aryl, CONH-heteroaryl, CONH-alkyl-heteroaryl, CONH-heterocycloalkyl, CONH-alkyl-heterocycloalkyl;
or
COO-alkyl, COO-cycloalkyl, COO-aryl, COO-alkyl-cycloalkyl, COO-alkyl-aryl, COO-heteroaryl, COO-alkyl-heteroaryl, COO-heterocycloalkyl, COO-alkyl-heterocycloalkyl;
or
SO₂-cycloalkyl, SO₂-aryl, SO₂-alkyl-cycloalkyl, SO₂-alkyl-aryl, SO₂-heteroaryl, SO₂-alkyl-heteroaryl, SO₂-heterocycloalkyl, SO₂-alkyl-heterocycloalkyl;
or
C(NCN)NH-alkyl, C(NCN)NH-cycloalkyl, C(NCN)NH-aryl, C(NCNNH)-alkyl-cycloalkyl, C(NCN) NH-alkyl-aryl, C(NCN)NH-heteroaryl, C(NCN)NH-alkyl-heteroaryl, C(NCN)NH-heterocycloalkyl, C(NCN)NH-alkyl-heterocycloalkyl;
or
C(NNO₂)NH-alkyl, C(NNO₂)NH-cycloalkyl, C(NNO₂)NH-aryl, C(NNO₂)NH-alkyl-cycloalkyl, C(NNO₂)NH-alkyl-aryl, C(NNO₂)NH-heteroaryl, C(NNO₂)NH-alkyl-heteroaryl, C(NNO₂)NH-heterocyloalkyl, C(NNO₂)NH-alkyl-heterocycloalkyl;
or
C(NH)NH-alkyl, C(NH)NH-cycloalkyl, C(NH)NH-aryl, C(NH)NH-alkyl-cycloalkyl, C(NH) NH-alkyl-aryl, C(NH)NH-heteroaryl, C(NH)NH-alkyl-heteroaryl, C(NH)NH-heterocycloalkyl, C(NH)NH-alkyl-heterocycloalkyl;
or
C(NH)NHCO-alkyl, C(NH)NHCO-cycloalkyl, C(NH)NHCO-aryl, C(NH)NHCO-alkyl-cycloalkyl, C(NH)NHCO-alkyl-aryl, C(NH)NHCO-heteroaryl, C(NH)NHCO-alkyl-heteroaryl, C(NH)NHCO-heterocylcloalkyl, C(NH)NHCO-alkyl-heterocycloalkyl;
or
C(NOR₆)NH-alkyl, C(NOR₆)NH-cycloalkyl, C(NOR₆)NH-aryl, C(NOR₆)NH-alkyl-cycloalkyl, C(NOR₆)NH-alkyl-aryl, C(NOR₆)NH-heteroaryl, C(NOR₆)NH-alkyl-heteroaryl, C(NOR₆)NH-heterocylcoalkyl, C(NOR₆)NH-alkyl-heterocycloalkyl;
R₅ is hydrogen or alkyl;
R₆ is hydrogen, alkyl, cycloalkyl, aryl, cycloalkylakyl, arylalkyl, heteroaryl, heteroarylalkyl, heterocycloalkyl or heterocycloalkylalkyl;
m is an integer of 0 to 2; and
n is an integer of 1 to 3;
or a pharmaceutically acceptable salt, enantiomer, diastereomer, isomer or tautomer thereof;
and a VEGF-R inhibitor (Compound B) of general formula (II): 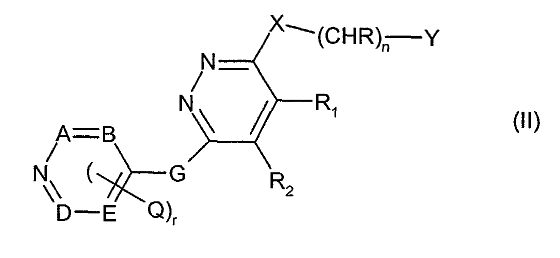 wherein:
R₁ and R₂ (i) are lower alkyl or (ii) together form a bridge of subformula I* 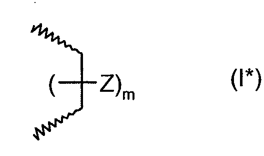 the binding being achieved via the two terminal carbon atoms, or (iii) R₁ and R₂ together form a bridge of subformula I** 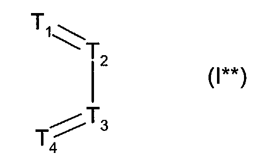
wherein one or two of the ring members T₁, T₂, T₃ and T₄ are nitrogen, and the others are in each case CH, and the binding is achieved via T₁ and T₄;
A, B, D, and E are in each case CH;
G is lower alkylene;
Q is methyl, which is bound to A, to D, or to A and D;
R is H or lower alkyl;
X is imino;
Y is phenyl, which is unsubstituted or substituted by one or two substituents independently of one another from the group comprising amino, lower alkanoylamino, halogen, lower alkyl, halogen-lower alkyl, hydroxy, lower alkoxy, phenyl-lower alkoxy, and cyano, or is pyridyl;
Z is amino, N-lower alkylamino, hydroxy-lower alkylamino, phenyl-lower alkylamino, N,N-dilower alkylamino, n-phenyl-lower alkyl-N-lower alkylamino, N, N-di-lower alkylphenylamino, lower alkanoylamino; or a substituent from the group comprising benzoylamino or phenyllower alkoxycarbonylamino, wherein the phenyl radical in each case is unsubstituted or especially substituted by nitro or amino, or by halogen, amino, N-lower alkylamino, N,N-dilower alkylamino, hydroxy, cyano, carboxy, lower alkoxycarbonyl, lower alkanoyl or carbamoyl; or is halogen;
r is 0 to 2;
n is 0 or 1;
m is 0 or 1; and,
the bonds **characterized by** a wavy line are in each case a double bond or in each case a single bond;
or a pharmaceutically acceptable salt, enantiomer, diastereomer, isomer or tautomer thereof;
and/or of general formula (IIa): 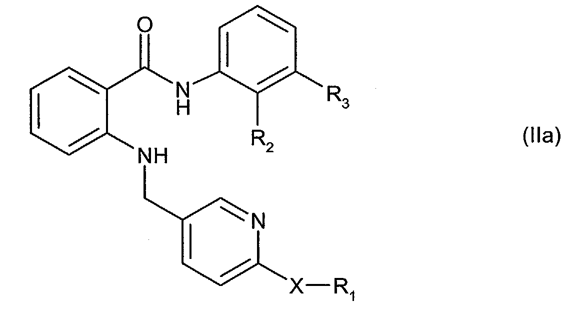 wherein:
R₁ is H or lower alkyl;
R₂ is H or lower alkyl;
R₃ is perfluoro lower alkyl; and
X is O or S,
or an N-oxide or a tautomer thereof,
or a pharmaceutically acceptable salt, enantiomer, diastereomer, isomer or tautomer thereof;
and/or of general formula (IIb): 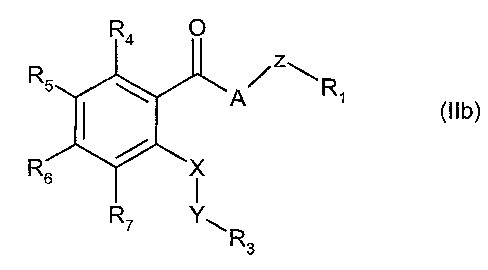 wherein:
A is NR₂
Z is NH, NR₁₀, N(R₁₀)-(CH₂)_{q} or the group 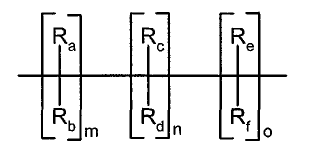 or A, Z and R₁ together form one of the following groups: 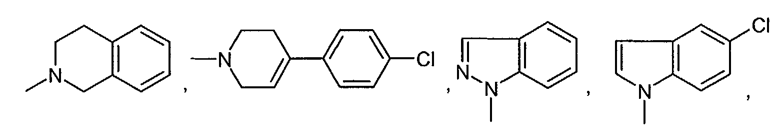 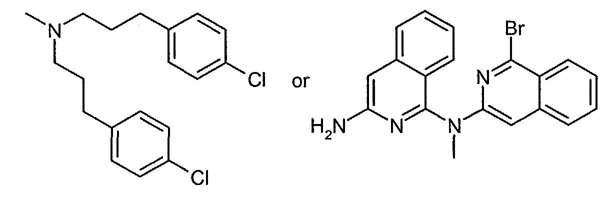
m, n and o are 0 - 3;
q is 1- 6;
Rₐ, R_{b}, R_{c}, R_{d}, Rₑ, R_{f}, independently of one another, are hydrogen or methyl or the group NR₁₀;
X is NR₉;
Y is CH₂;
R₁ is phenyl, pyridyl, 5-chloro-2,3-dihyroindenyl, 2,3-dihydroindenyl, thienyl, 6-fluoro-1 H-indol-3-yl, naphthyl, 1,2,3,4-tetrahydronaphthyl, benzo-1,2,5-oxadiazole or 6,7-dimethoxy-1,2,3,4-tetrahydro-2-naphthal or is phenyl or pyridyl that is substituted in one or more places with C₁-C₄ alkyl, C₁-C₄ alkoxy, hydroxy, halogen, or trifluoromethyl or is one of the following groups: 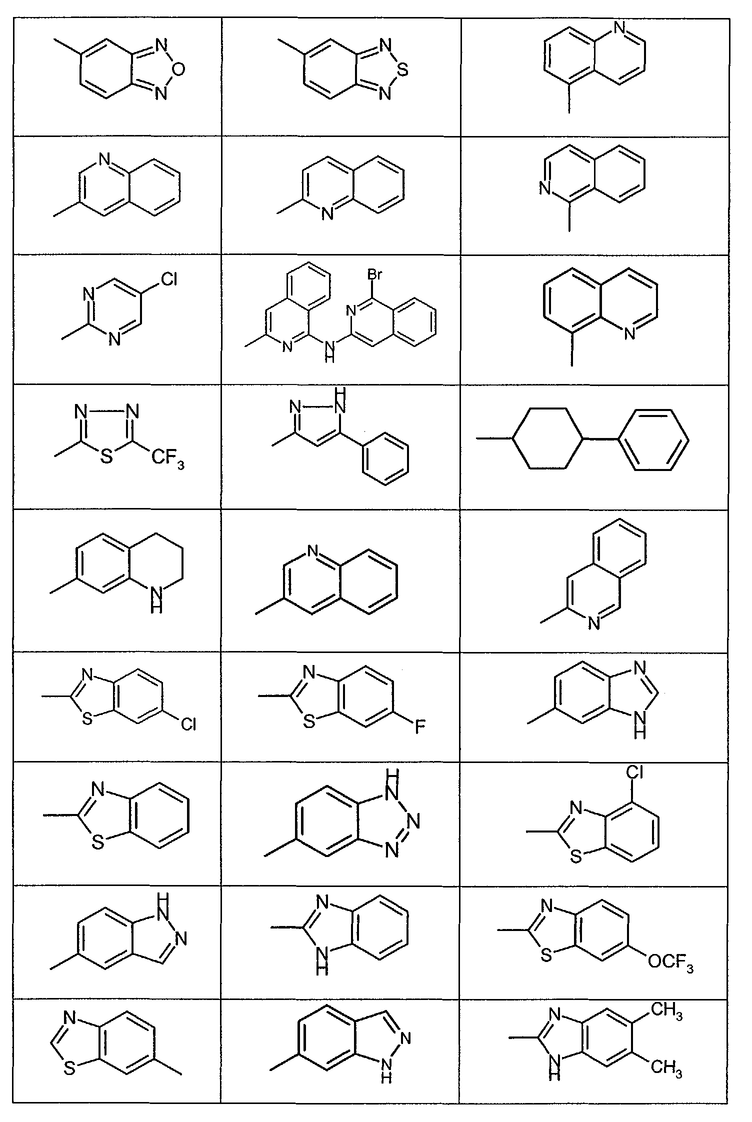 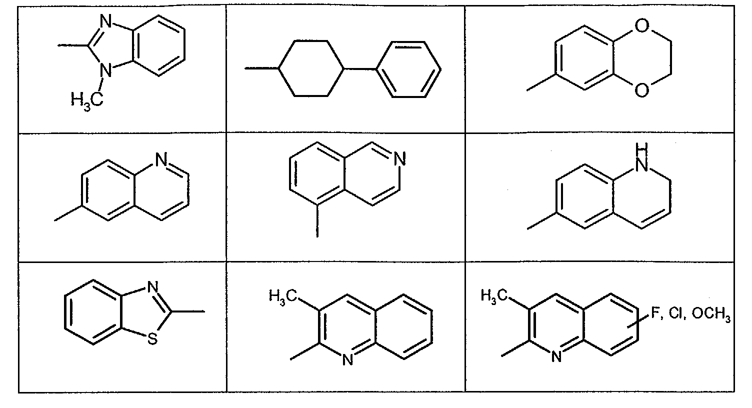
whereby phenyl or substituted phenyl or naphthyl is not directly bonded to the NR₂ group in the meaning of A;
R₂ is H or CH₃;
R₃ is pyridyl or phenyl, pyridyl or 1,2,3,4-tetrahydronaphthyl that is substituted in one or more places with hydroxy, halogen, methyl or methoxy, or is one of the following groups: 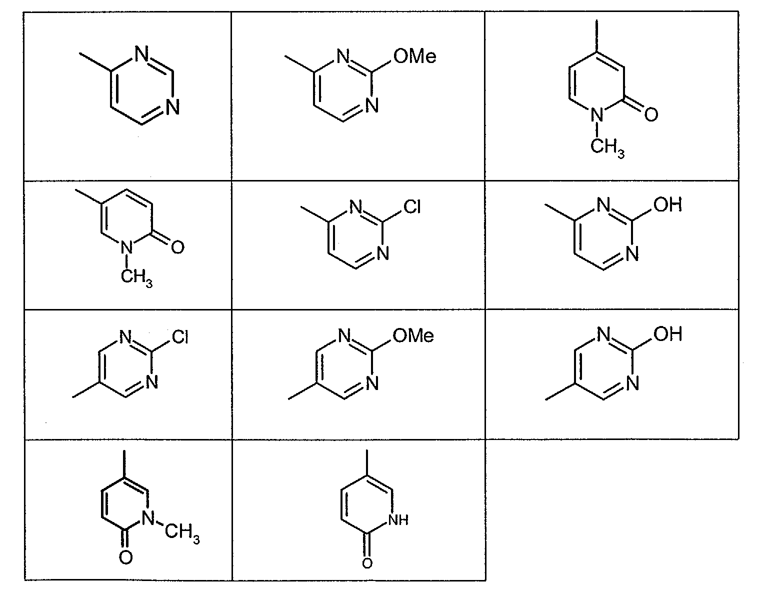
R₅ and R₆, independently of one another, are hydrogen, halogen, methyl, methoxy or trifluoromethyl;
R₄ and R₇, independently of one another, are hydrogen or halogen;
R₉ is H or C₁-C₆ alkyl;
R₁₀ is H or methyl;
or a pharmaceutically acceptable salt, enantiomer, diastereomer, isomer or tautomer thereof.

2. A combination as claimed in Claim 1, wherein in formula (I):
R₁ and R₂ are independently hydrogen, fluorine or alkyl;
R₃ is 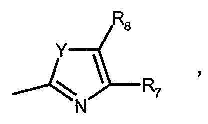 wherein Y is 0, S or NR₉;
R₄ is alkyl, cycloalkyl, aryl, cycloalkylalkyl, arylalkyl, heteroaryl, heteroarylalkyl, heterocycloalkyl, heterocycloalkylalkyl;
or
CO-alkyl, CO-cycloalkyl, CO-aryl,CO-alkyl-cycloalkyl, CO-alkyl-aryl, CO-heteroaryl,CO-alkyl-heteroaryl, CO-heterocycloalkyl, CO-alkyl-heterocycloalkyl; or CONH-alkyl, CONH-cycloalkyl, CONH-aryl, CONH-alkyl-cycloalkyl, CONH-alkyl-aryl, CONH-heteroaryl, CONH-alkyl-heteroaryl,CONH-heterocycloalkyl, CONH-alkyl-heterocycloalkyl;
or
COO-alkyl, COO-cycloalkyl, COO-aryl, COO-alkyl-cycloalkyl, COO-alkyl-aryl, COO-heteroaryl, COO-alkyl-heteroaryl, COO-heterocycloalkyl,COO-alkyl-heterocycloalkyl;
or
SO₂-cycloalkyl, SO₂-aryl, SO₂-alkyl-cycloalkyl, SO₂-alkyl-aryl, SO₂-heteroaryl, SO₂-alkyl-heteroaryl, SO₂-heterocycloalkyl, SO₂-alkyl-heterocycloalkyl;
or
C(NCN)NH-alkyl, C(NCN)NH-cycloalkyl, C(NCN)NH-aryl, C(NCNNH)-alkyl-cycloalkyl, C(NCN)NH-alkyl-aryl, C(NCN)NH-heteroaryl, C(NCN)NH-alkyl-heteroaryl, C(NCN)NH-heterocycloalkyl, C(NCN)NH-alkyl-heterocycloalkyl;
or
C(NNO₂)NH-alkyl, C(NNO₂)NH-cycloalkyl, C(NNO₂)NH-aryl, C(NNO₂)NH-alkyl-cycloalkyl, C(NNO₂)NH-alkyl-aryl, C(NNO₂)NH-heteroaryl, C(NNO₂)NH-alkyl-heteroaryl, C(NNO₂)NH-heterocyloalkyl, C(NNO₂)NH-alkyl-heterocycloalkyl;
or
C(NH)NH-alkyl, C(NH)NH-cycloalkyl, C(NH)NH-aryl, C(NH)NH-alkyl-cycloalkyl, C(NH)NH-alkyl-aryl, C(NH)NH-heteroaryl, C(NH)NH-alkyl-heteroaryl, C(NH)NH-heterocycloalkyl, C(NH)NH-alkyl-heterocycloalkyl;
or
C(NH)NHCO-alkyl, C(NH)NHCO-cycloalkyl, C(NH)NHCO-aryl, C(NH)NHCO-alkyl-cycloalkyl, C(NH)NHCO-alkyl-aryl, C(NH)NHCO-heteroaryl, C(NH)NHCO-alkyl-heteroaryl, C(NH)NHCO-heterocylcloalkyl, C(NH)NHCO-alkyl-heterocycloalkyl;
or
C(NOR₆)NH-alkyl, C(NOR₆)NH-cycloalkyl, C(NOR₆)NH-aryl, C(NOR₆)NH-alkyl-cycloalkyl, C(NOR₆)NH-alkyl-aryl, C(NOR₆)NH-heteroaryl, C(NOR₆)NH-alkyl-heteroaryl, C(NOR₆) NH-heterocylcoalkyl, C(NOR₆)NH-alkyl-heterocycloalkyl;
R₅ is hydrogen;
R₆ is hydrogen, alkyl, cycloalkyl, aryl, cycloalkylakyl, arylalkyl, heteroaryl, heteroarylalkyl, heterocycloalkyl or heterocycloalkylalkyl;
R₇ and R₈ are independently hydrogen, alkyl, cycloalkyl, aryl, alkylcycloalkyl, alkylaryl, heteroaryl, alkylheteroaryl, heterocycloalkyl, alkylheterocycloalkyl or halogen;
R₉ is H or alkyl;
m is the integer 0; and
n is the integer 1.

3. A combination as claimed in Claim 2, wherein:
R₁ is H;
R₂ is H, F or alkyl;
R₃ is a substituted oxazole having the configuration : 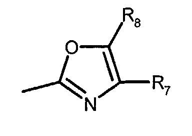
R₄ is CO-alkyl, CO-alkyl-aryl, CO-cycloalkyl, CO-alkyl-heteroaryl, CO-alkyl-heteroalkyl, CO-heterocycloalkyl, CO-alkyl-heterocycloalkyl, aryl, arylalkyl, heteroaryl, heteroarylalkyl;
R₅ is H;
R₇ is H;
R₈ is an alkyl group, such as tert-butyl;
m is the integer 0; and
n is the integer 1.

4. A combination as claimed in Claim 3, wherein in formula (I):
R₂ is H;
R₄ is CO-heterocycloalkyl or CO-cycloalkyl;
R₈ is tert-butyl.

5. A combination as claimed in Claim 1, wherein the compound of formula (I) is at least one of the following compounds:
N-[5-[[[5-(1,1-dimethylethyl)-2-oxazolyl] methyl]thio]-2-thiazolyl]-4-piperidinecarboxamide;
(±)-N-[5-[[[5-(1,1-dimethylethyl)-2-oxazolyl] methyl]thio]-2-thiazolyl]-3-piperidinecarboxamide;
(±)-1-(2,3-dihydroxypropyl)-N-[5-[[[5-(1,1-dimethylethyl)-2-oxazolyl]-methyl]thio]-2-thiazolyl]-4-piperidinecarboxamide;
N-[5-[[[5-(1,1-dimethylethyl)-2-oxazolyl] methyl]thio]-2-thiazolyl]-1-(1-methylethyl)-4-piperidinecarboxamide;
1-cyclopropyl-N-[5-[[[5-(1,1-dimethylethyl)-2-oxazolyl]methyl]thio]-2-thiazolyl]-4-piperidinecarboxamide;
N-[5-[[[5-(1,1-dimethylethyl)-2-oxazolyl] methyl]thio]-2-thiazolyl]-1-(2-hydroxyethyl)-4-piperidinecarboxamide;
(R)-N-[5-[[[5-(1,1-dimethylethyl)-2-oxazolyl]methyl]thio]-2-thiazolyl]-3-piperidinecarboxamide;
(S)-N-[5-[[[5-(1,1-dimethylethyl)-2-oxazolyl]methyl]thio]-2-thiazolyl]-3-piperidinecarboxamide;
cis-4-amino-N-[5-[[[5-(1,1-dimethylethyl)-2-oxazolyl] methyl]thio]-2-thiazolyl] cyclohexylcarboxamide;
trans-4-amino-N-[5-[[[5-(1,1-dimethylethyl)-2-oxazolyl] methyl]thio]-2-thiazolyl] cyclohexylcarboxamide; or
N-[5-[1[5-(1,1-Dimethylethyl)-2-oxazolyllmethyllthiol-2-thiazolyll-4-piperidinecarboxamide;
or a pharmaceutically acceptable salt, enantiomer, diastereomer, isomer or tautomer thereof.

6. A combination as claimed in Claim 1, wherein the compound of formula (I) is: N-[5-[[[5-(1,1-dimethylethyl)-2-oxazolyl] methyl]thio]-2-thiazolyl]-4-piperidinecarboxamide or a pharmaceutically acceptable salt, enantiomer, diastereomer, isomer or tautomer thereof.

7. A combination as claimed in any one of Claims 1 to 6, wherein Compound B comprises a compound of general formula (II): 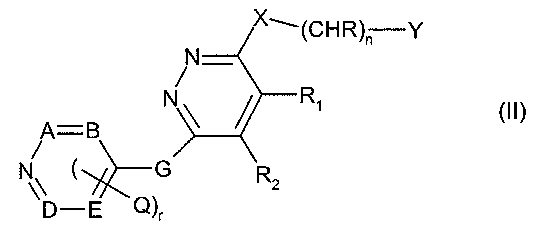 wherein:
R₁ and R₂ (i) are lower alkyl or (ii) together form a bridge of subformula I* 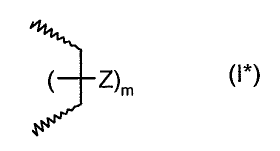 the binding being achieved via the two terminal carbon atoms, or (iii) R₁ and R₂ together form a bridge of subformula I** 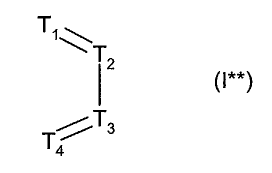
wherein one or two of the ring members T₁, T₂, T₃ and T₄ are nitrogen, and the others are in each case CH, and the binding is achieved via T₁ and T₄;
A, B, D, and E are in each case CH;
G is lower alkylene;
Q is methyl, which is bound to A, to D, or to A and D;
R is H or lower alkyl;
X is imino;
Y is phenyl, which is unsubstituted or substituted by one or two substituents independently of one another from the group comprising amino, lower alkanoylamino, halogen, lower alkyl, halogen-lower alkyl, hydroxy, lower alkoxy, phenyl-lower alkoxy, and cyano, or is pyridyl;
Z is amino, N-lower alkylamino, hydroxy-lower alkylamino, phenyl-lower alkylamino, N,N-dilower alkylamino, n-phenyl-lower alkyl-N-lower alkylamino, N, N-di-lower alkylphenylamino, lower alkanoylamino; or a substituent from the group comprising benzoylamino or phenyllower alkoxycarbonylamino, wherein the phenyl radical in each case is unsubstituted or especially substituted by nitro or amino, or by halogen, amino, N-lower alkylamino, N,N-dilower alkylamino, hydroxy, cyano, carboxy, lower alkoxycarbonyl, lower alkanoyl or carbamoyl; or is halogen;
r is 0 to 2;
n is 0 or 1;
m is 0 or 1; and,
the bonds **characterized by** a wavy line are in each case a double bond or in each case a single bond;
or a pharmaceutically acceptable salt, enantiomer, diastereomer, isomer or tautomer thereof.

8. A combination as claimed in Claim 7, wherein in formula (II):
R₁ and R₂ together form a bridge of subformula I*, the binding being achieved via the two terminal carbon atoms;
A, B, D, and E are in each case CH;
G is methylene;
R is H;
X is imino;
Y is phenyl, which is substituted or unsubstituted by one or two substituents independently of one another from the group comprising amino, lower alkanoylamino, halogen, lower alkyl, halogen-lower alkyl, hydroxy, lower alkoxy, phenyl-lower alkoxy, and cyano;
r is 0;
n is 0 or 1;
m is 0; and,
the bonds **characterized by** a wavy line are double bonds.

9. A combination as claimed in any one of Claims 1 to 6, wherein Compound B comprises a compound of general formula (IIa): 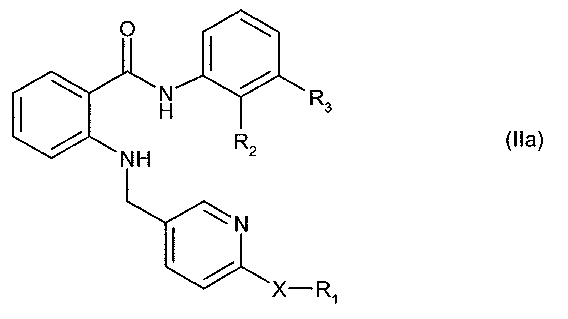 wherein:
R₁ is H or lower alkyl;
R₂ is H or lower alkyl;
R₃ is perfluoro lower alkyl; and
X is O or S,
or an N-oxide or a tautomer thereof,
or a pharmaceutically acceptable salt, enantiomer, diastereomer, isomer or tautomer thereof.

10. A combination as claimed in Claim 9, wherein in formula (IIa):
R₁ is H or methyl;
R₂ is H or methyl;
R₃ is trifluoromethyl; and
X is O.

11. A combination as claimed in any one of Claims 1 to 6, wherein Compound B comprises a compound of general formula (IIb): 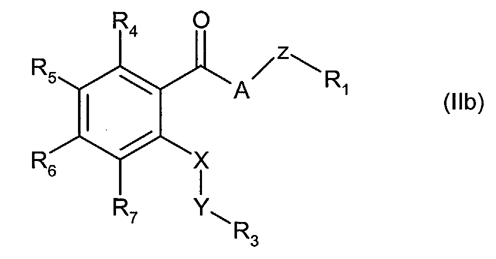 wherein:
A is NR₂
Z is NH, NR₁₀, N(R₁₀)-(CH₂)_{q} or the group 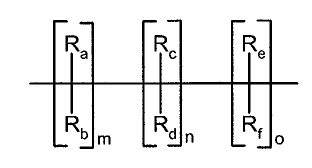 or A, Z and R₁ together form one of the following groups: 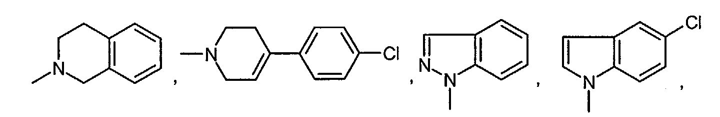 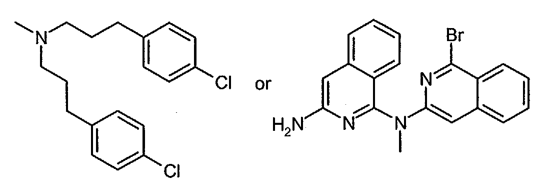
m, n and o are 0 - 3;
q is 1-6;
Rₐ, R_{b}, R_{c}, R_{d}, Rₑ, R_{f}, independently of one another, are hydrogen or methyl or the group NR₁₀;
X is NR₉;
Y is CH₂;
R₁ is phenyl, pyridyl, 5-chloro-2,3-dihyroindenyl, 2,3-dihydroindenyl, thienyl, 6-fluoro-1 H-indol-3-yl, naphthyl, 1,2,3,4-tetrahydronaphthyl, benzo-1,2,5-oxadiazole or 6,7-dimethoxy-1,2,3,4-tetrahydro-2-naphthal or is phenyl or pyridyl that is substituted in one or more places with C₁-C₄ alkyl, C₁-C₄ alkoxy, hydroxy, halogen, or trifluoromethyl or is one of the following groups: 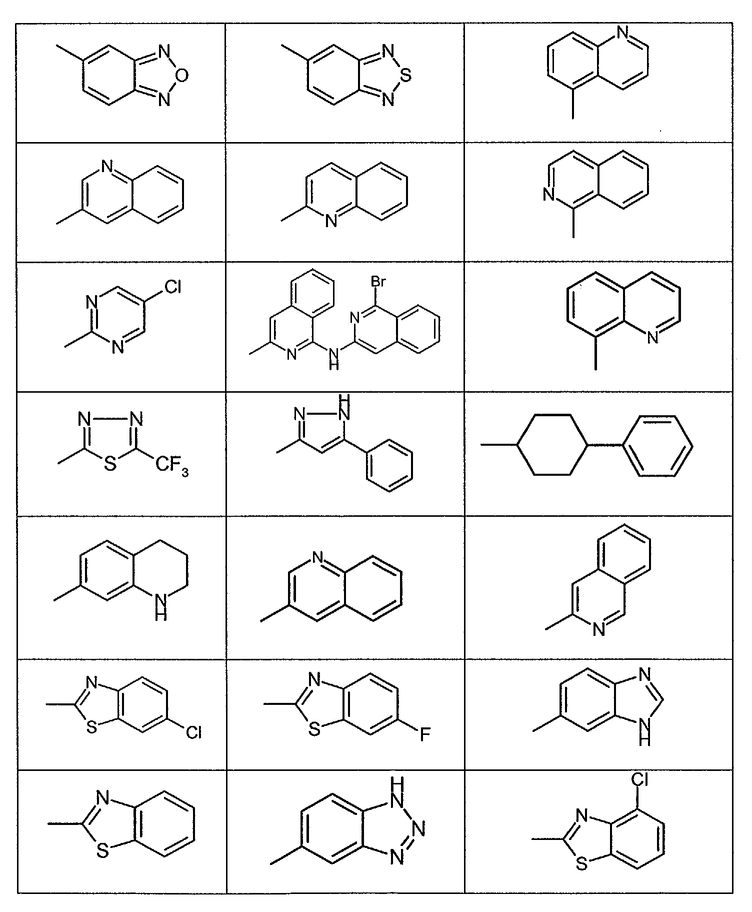 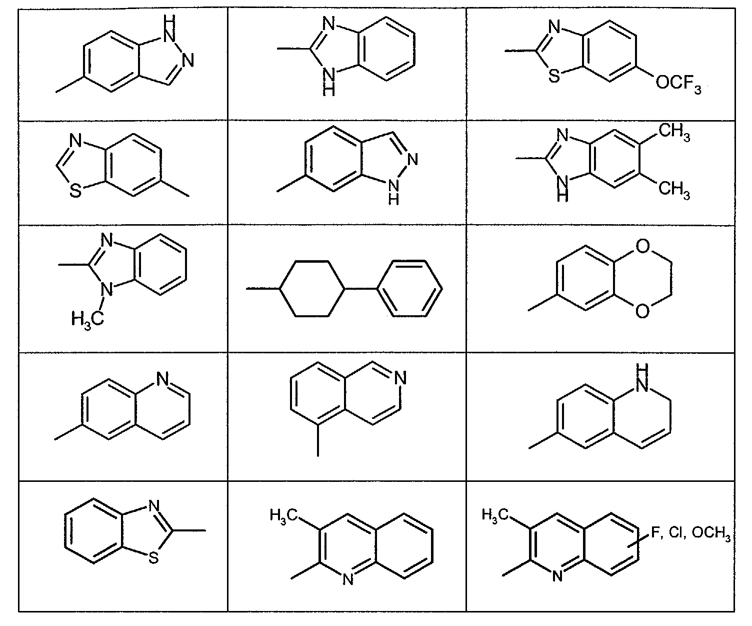 whereby phenyl or substituted phenyl or naphthyl is not directly bonded to the NR₂ group in the meaning of A;
R₂ is H or CH₃;
R₃ is pyridyl or phenyl, pyridyl or 1,2,3,4-tetrahydronaphthyl that is substituted in one or more places with hydroxy, halogen, methyl or methoxy, or is one of the following groups: 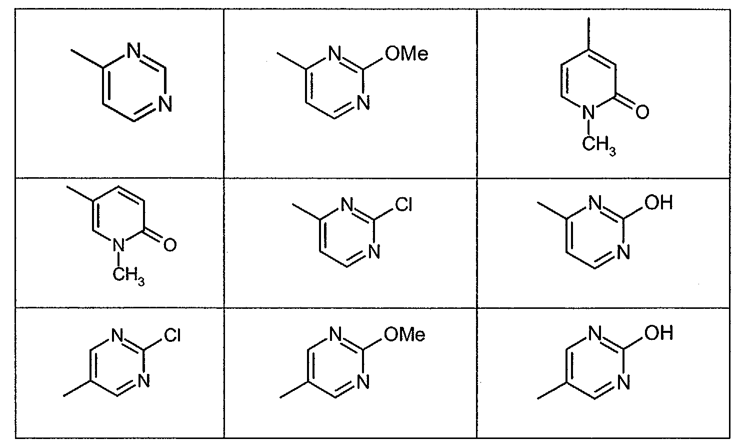 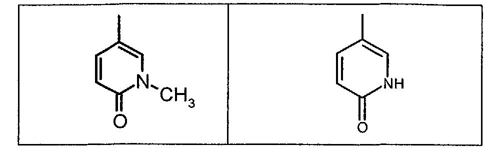
R₅ and R₆, independently of one another, are hydrogen, halogen, methyl, methoxy or trifluoromethyl;
R₄ and R₇, independently of one another, are hydrogen or halogen;
R₉ is H or C₁-C₆ alkyl;
R₁₀ is H or methyl;
or a pharmaceutically acceptable salt, enantiomer, diastereomer, isomer or tautomer thereof.

12. A combination as claimed in Claim 11, wherein in formula (IIb):
Z is the group 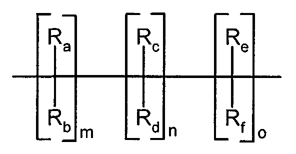 and m, n, and o are 0;
or A, Z and R₁ together form one of the following groups: 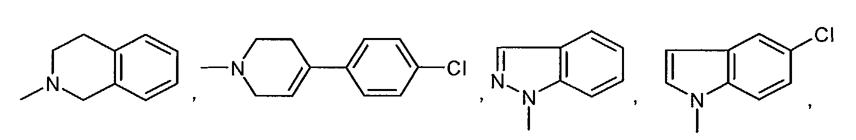 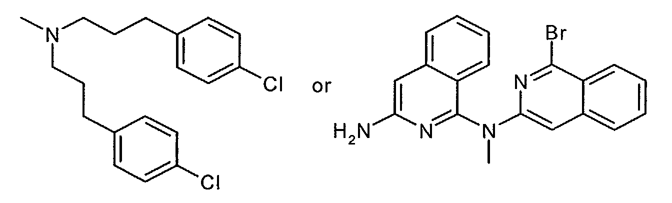
R₉ is H; and
R₄, R₅, R₆, and R₇, independently of one another are H or halogen.

13. A combination as claimed in any one of Claims 1 to 6 wherein Compound B is at least one of the following three compounds:

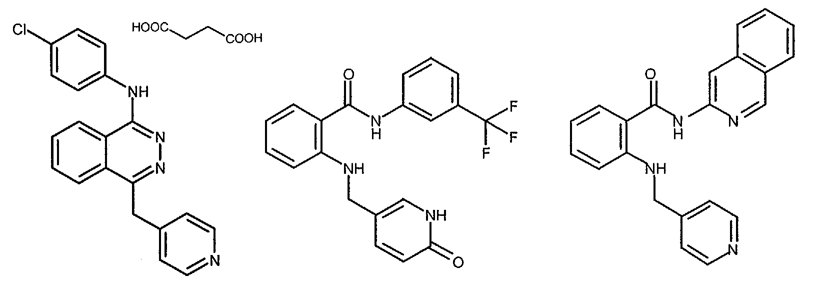

14. A combination as claimed in Claim 13, wherein Compound B is:

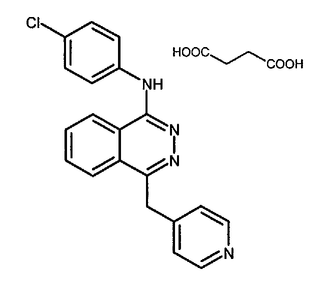

15. A combination as claimed in Claim 13, wherein Compound B is:

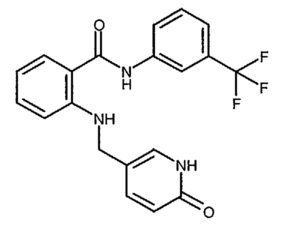

16. A combination as claimed in Claim 13, wherein Compound B is:

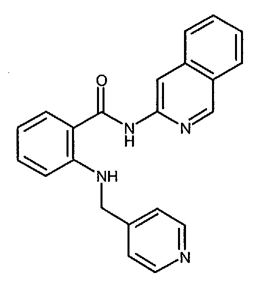

17. A combination according to any one of Claims 1 to 16 for use in a method for the treatment of the human or animal body.

18. A combination according to any one of Claims 1 to 16, which is suitable for administration to a warm-blooded animal, especially suffering from a disease which responds to an inhibition of CDK and VEGF-R, comprising an effective amount of the combination, together with at least one pharmaceutically acceptable adjuvant, excipient or carrier.

19. Use of the combination as claimed in any one of Claims 1 to 16 in the manufacture of a medicament for the treatment or prophylaxis of cancer and tumor diseases.

20. Use according to Claim 19, with at least one pharmaceutically acceptable adjuvant, excipient or carrier.

21. Use of the combination as claimed in any one of Claims 1 to 16 in the treatment of auto-immune diseases such as psoriasis, alopecia, multiple sclerosis, chemotherapy-induced alopecia and mucositis; cardiovascular diseases such as stenoses, arterioscleroses and restenoses; infectious diseases caused by unicellular parasites, such as trypanosoma, toxoplasma or plasmodium, or produced by fungi; nephrological diseases, such as glomerulonephritis; chronic neurodegenerative diseases, such as Huntington's disease, amyotropic lateral sclerosis, Parkinson's disease, AIDS dementia and Alzheimer's disease; acute neurodegenerative diseases, such as ischemias of the brain and neurotraumas; viral infections, such as cytomegalic infections, herpes, Hepatitis B and C, and HIV diseases.

22. Use of the combination as claimed in any one of Claims 1 to 16 in the treatment of haemeangioma, angiofribroma, diseases of the eyes, such as diabetic retinopathie, neovascular glaucoma, diseases of the kidney, such as glomerulonephritis, diabetic nephropatic diseases, malignant nephrosclerosis, thrombotic microangiopatic syndrome, disposes of transplants and glomerulopathy, fibrotic diseases, such as liver cirrhosis, mesangialic cell proliferative diseases and artheriosclerosis, injury of the nervous tissues, for inhibition of reocclusion of vascular systems after balloon catheter treatment, for artificial limbs, or after insert of mechanically devices for keeping open of vasculature, such as stents.

23. Use of the combination as claimed in any one of Claims 1 to 16 in the treatment of injury of the nervous tissues.

24. Use of the combination as claimed in any one of Claims 1 to 16 in the suppression of oedema.

25. Use of the combination as claimed in any one of Claims 1 to 16 for the preparation of a pharmaceutical product for the treatment of a disease.

26. Use according to Claim 25, for the preparation of a pharmaceutical product for the treatment of a disease which responds to an inhibition of CDK and VEGF-R.

27. Use of the combination as claimed in any one of Claims 1 to 16 for the treatment of a disease which responds to an inhibition of CDK and VEGF-R.

28. A method of treatment of a disease which responds to an inhibition of CDK and VEGF-R, which comprises administering a combination according to any one of Claims 1 to 16, in a quantity therapeutically effective against the said diseases, to a warm-blooded animal.

29. A method of treatment or prophylaxis of a cancer or tumor disease in a person or animal suffering from or susceptible to such a disease, which method comprises administering to the person or animal a therapeutically effective amount of a combination according to any one of the Claims 1 to 16.

30. A pharmaceutical composition comprising a combination according to any one of Claims 1 to 16 in association with a pharmaceutically acceptable adjuvant, diluent or carrier.

31. A kit comprising a combination according to any of Claims 1 to 16, wherein at least one compound of general formula (I) and at least one compound of general formula (II), (IIa) or (IIb) are used as a combined preparation simultaneously, separately or sequentially.
